(19) Europäisches Patentamt
European Patent Office
Office européen des brevets

(11) Numéro de publication: **0 104 964**
**B1**

(12) # FASCICULE DE BREVET EUROPEEN

(45) Date de publication du fascicule du brevet: **01.07.87**

(51) Int. Cl.$^4$: **C 07 K 7/02,** A 61 K 37/02

(21) Numéro de dépôt: **83401641.2**

(22) Date de dépôt: **10.08.83**

(54) **Dérivés peptidiques inhibiteurs de protéases acides.**

(30) Priorité: **17.08.82 FR 8214219**

(43) Date de publication de la demande:
**04.04.84 Bulletin 84/14**

(45) Mention de la délivrance du brevet:
**01.07.87 Bulletin 87/27**

(84) Etats contractants désignés:
**AT BE CH DE FR GB IT LI LU NL SE**

(56) Documents cités:
**FR-A-2 262 532**

**H. UMEZAWA et al.: "BIOACTIVE PEPTIDES PRODUCED BY MICROORGANISMS", John Wiley & Sons, NEW YORK (US)**

**CHEMICAL ABSTRACTS, vol. 90, 1979, page 16, résumé no. 161943h, COLUMBUS, Ohio (US) W.S. LIU et al.: "Synthesis of all the stereoisomers of statine (4-amino-3-hydroxy-6-methylheptanoic acid). Inhibition of pepsin activity by N-carbobenzoxy-L-valyl-L-valyl-statine derived from the four stereoisomers"**

(73) Titulaire: **SANOFI, société anonyme
40, Avenue George V
F-75008 Paris (FR)**

(73) Titulaire: **INSTITUT NATIONAL DE LA SANTE ET DE LA RECHERCHE MEDICALE (INSERM)
101, rue de Tolbiac
F-75654 Paris Cedex 13 (FR)**

(72) Inventeur: **Cazaubon, Catherine
9 rue Pierre Boissier
F-34000 Montpellier (FR)**
Inventeur: **Diaz, Joseph
19 rue du Pradas
F-34470 Perols (FR)**
Inventeur: **Gagnol, Jean-Pierre
Place de l'Eglise
F-34380 Saint Martin de Londres (FR)**
Inventeur: **Guegan, Rémy
355 Chemin du Thym
F-34170 Castelnau Le -Lez (FR)**
Inventeur: **Castro, Bernard
7 rue Hélène Boucher
F-34470 Perols (FR)**
Inventeur: **Corvol, Pierre
88 rue de Sèvres
F-75007 Paris (FR)**
Inventeur: **Evin, Geneviève
24 rue Vergniaud
F-75013 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**EP 0 104 964 B1**

(74) Mandataire: **Gillard, Marie-Louise et al**
**Cabinet Beau de Loménie 55, Rue d'Amsterdam**
**F-75008 Paris (FR)**

**Description**

En 1970, UMEZAWA a isolé, à partir d'une culture de Streptomyces, un pentapeptide désigné sous le nom de Pepstatine dont la structure a été établie ultérieurement et répond à la formule Isovaléryl - L - valyl - L - valyl - Stalyl - L - alanyl - Statine, dans laquelle on désigne sous le nom de statine un acide aminé non usuel, l'acide (3S, 4S) amino - 4 - hydroxy - 3 - méthyl - 6 heptanoïque.

Il a été montré que la pepstatine est un inhibiteur de protéases acides et agit en particulier sur la pepsine et la rénine. Spécialement, la rénine, enzyme d'origine rénale, intervient dans la séquence angiotensinogène, angiotensine I, angiotensine II au niveau de la transformation de l'angiotensinogène en angiotensine.

L'angiotensine étant responsable de l'élévation de la tension artérielle, on a envisagé d'utiliser la pepstatine pour lutter contre l'hypertension chez l'homme.

Toutefois, la pepstatine agissant sur l'ensemble des protéases acides, son emploi en thérapeutique s'est avéré difficile.

Quelques dérivés de la pepstatine ont été décrits dans la littérature scientifique. Il apparaît toutefois que leur niveau d'activité est en général relativement modeste et leur sélectivité faible.

La présente invention a pour objet la préparation de nouveaux dérivés de la pepstatine présentant un niveau d'activité élevé sur l'inhibition de la résine et de la pepsine.

Les composés obtenus selon l'invention répondent à la formule générale:

$$R—X—Y—Statyl_1\text{-Ala-}Statyl_2\text{-}R' \qquad (1)$$

dans laquelle on désigne par $Statyl_1$ le radical dérivant de l'aminoacide statine, à savoir:

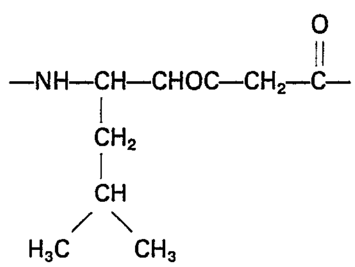

isomère 3S, 4S

et par $Statyl_2$ le radical statyl dérivant du même aminoacide, qu'il s'agisse de l'isomère 3S, 4S ou de l'isomère 3R, 4S

— R désigne un atome d'hydrogène ou un groupement acylant fixé sur le groupe amino terminal de l'aminoacide X,
— X et Y identiques ou différents désignent des acides aminés de configuration L ou D lorsqu'il existe un centre d'asymétrie dans la molécule, et éventuellement protégés dans leur chaîne latérale, lesdits acides aminés étant choisis parmi les acides aminés suivants:
— X : Phénylalanine, Tryptophane, Histidine, Boc Histidine, Tyrosine, Proline, Isoleucine;
— Y : Phénylalanine, Tryptophane, Histidine, Tyrosine, Proline, Leucine, Isoleucine, Valine, Glycine;
— R': représente OH, O-alkyle inférieur.

Plus particulièrement, R peut représenter les groupements acétyl (Ac), isovaléryl (iVa), octyl, t.butoxycarbonyl (Boc), adamantyloxycarbonyl (A doc), benzyloxycarbonyl (Z), (benzyloxyarbonyl) β-amino-éthylsulfonyl (Z-Tau), (t-butyloxycarbonyl) β-amino-éthylsulfonyl (Boc-Tau), phénylsulfonyl, benzylsulfonyl, phényl-3 propionyl.

— X et Y identiques ou différents désignent des acides aminés de configuration L ou D lorsqu'il existe un centre d'asymétrie dans la molécule, et éventuellement protégés dans leur chaîne latérale.

Les produits obtenus selon l'invention comprennent également les sels pharmaceutiquement acceptables que peuvent éventuellement fournir les composés de formule (1) lorsque R' représente OH.

Les produits sont selon l'invention préparés selon les méthodes habituelles de la chimie des peptides. En particulier, ils peuvent être préparés par un procédé étape par étape dit "procédé Stepwise" à partir du C terminal.

Le produit de départ est un ester d'alkyle inférieur de la statine sur lequel est condensé l'acide aminé suivant.

Après libération de la fonction amine du dipeptide, on procède à l'élongation de la chaîne peptidique par couplage de l'acide aminé suivant convenablement protégé. Chaque phase de couplage est suivie d'une opération sélective de libération de l'amine qui va entrer en réaction lors de la création de la liaison peptidique suivante.

Les différentes opérations de couplage sont effectuées soit en utilisant un ester activé de l'aminoacide à coupler, soit en utilisant l'aminoacide N protégé lui-même en présence de dicyclohexylcarbodiimide.

Les phases de déprotection sélective de l'amine sont effectuées selon la nature du groupe protecteur utilisé, soit par hydrogénolyse, soit par hydrolyse en milieu acide fort tel que l'acide trifluoracétique.

Enfin, lorsque l'acide aminé à introduire dans la séquence possède dans sa chaîne latérale une fonction susceptible de réagir (c'est notamment le cas de l'histidine), il convient de bloquer celle-ci par un groupe protecteur convenable que l'on élimine ultérieurement.

Enfin, les peptides (I) sous forme acide (R'=H) peuvent être obtenus à partir des esters correspondants par saponification en milieu alcalin dilué.

Les exemples suivants non limitatifs, sont donnés à titre d'illustration de la présente invention.

Dans tous ces exemples, les abréviations suivantes seront utilisées:

Acides aminés et groupes protecteurs ou activateurs

Ces abréviations sont en accord avec celles indiquées par la Commission de Nomenclature de l'IUPAC-IUB section Biochimie.

Acides aminés:

Ala: Alanine
His: Histidine
Ile: Isoleucine
Leu: Leucine
Phe: Phénylalanine
Pro: Proline
Trp: Tryptophane
Tyr: Tyrosine
Val: Valine
Nle: Norlencine
Nva: Norvaline
Tau: Taurine
Sta: Statine
iso Sta: isoStatine (isomère 3R, 4S)

Ces acides aminés sont, sauf indication contraire, de configuration L.
Ceux de la série D sont indiqués par (D) précédant l'abréviation.

Groupes protecteurs et activateurs

Ac: Acétyl
Adoc: Adamentyloxycarbonyl
Boc: T. butyloxycarbonyl
HONSu: N-hydroxysuccinimide
OEt: Ester éthylique
OMe: Ester méthylique
ONp: Ester p nitrophénylique
ONSu: Ester de N-hydroxysuccinimide
OTcp: Ester trichloro-2,4,5 phénylique
i Va: Isovaléryl
Z: Benzoyloxycarbonyl.

De plus nois utiliserons les abréviations suivantes:

AcOEt: Acétate d'éthyle
AcOH: Acide acétique
Bop: Hexafluorophosphate de benzotriazolyloxy tris diméthylamino phosphonium
CCM: Chromatographie en couche mince
DCCI: Dicyclohexylcarbodiimide
DCHA: Dicyclohexylamine
DCU: Dicyclohexylurée
DIPEA: Diisopropyléthylamine
DMF: Diméthylformamide
DMSO: Diméthylsulfoxyde
Ether: Ether éthylique
HOBt: hydroxy-1 benzotriazole
$KHSO_4$—$K_2SO_4$: Solution aqueuse contenant 16,6 g de bisulfate de potassium et 33,3 g de sulfate de potassium pour 1 litre
MeOH: Méthanol
NEM: N-éthyl morpholine
TA: Température ambiante
TFA: Acide trifluoracétique

Enfin, les spectres de résonance magnétique nucléaire ont été enregistrés à 250 MHz en solution dans le diméthylsulfoxyde, l'étalon interne étant l'hexaméthyldisiloxane.

Les abréviations suivantes sont utilisées: s: singulet
d: doublet
m: multiplet ou massif.

Exemple 1

Z-Phe-Phe-Sta-Ala-Sta-OMe (SR 41320)

1. Z-Ala-Sta-OMe

Dissoudre à température ambiante dans 35 ml de DMF, 1,44 g de H-Sta-OMe, TFA, 595 mg de NEM, 2,06 g de Z-Ala-OTcp et 1,29 g de HOBt. Ajuster le pH à 6—7 au papier pH, si nécessaire, par NEM et agiter à température ambiante durant 48 h. Evaporer le DMF au bain-marie à 40° sous 0,01 mm. Reprendre l'huile résiduelle dans 50 ml d'AcOEt et laver celui-ci successivement avec $KHSO_4$—$K_2SO_4$ 5%, $NaCl/H_2O$, $NaHCO_3$ 5%, $NaCl/H_2O$. Sécher sur $MgSO_4$ et évaporer le solvant.

Reprendre dans l'éther, un solide apparaît. Laisser une heure au réfrigérateur; essorer; sécher.

Rendement: 1,46 g (86 %); Fc: 117—120°C.

2. H-Ala-Sta-OMe

Dissoudre 1,46 g de Z-Ala-Sta-OMe dans 20 ml de MeOH, puis à température ambiante, ajouter 1,03 g de formiate d'ammonium, agiter jusqu'à dissolution, puis ajouter 400 mg de Pd/C 10% et agiter 5 min à température ambiante. Après 5 min, un contrôle en CCM montre la disparition du produit de départ. Le pH est de 8 au papier pH. Filtrer l'ensemble sur célite et évaporer le solvant. Filtrer le méthanol sur une colonne de résine Amberlite IR 45 ($O\bar{H}$) et évaporer le méthanol.

Reprendre dans l'éther et évaporer à sec. Dissoudre dans du chlorure de méthylène, filtrer l'insoluble et évaporer à sec; nous obtenons une poudre blanche.

Rendement: 810 mg (75%); Fc: 123—134°C.

3. Boc-Sta-Ala-Sta-OMe

Dissoudre successivement dans 50 ml de chlorure de méthylène à température ambiante 960 mg de H-Ala-Sta-OMe, 1 g de Boc-Sta-OH, 420 mg de HONSu, 750 mg de DCCI. Très rapidement un précipité blanc apparaît. Agiter à température ambiante, Après 7 h, filtrer la DCU formée et laver celle-ci avec du chlorure de méthylène. Laver la solution organique successivement avec $KHSO_4$—$K_2SO_4$, (5% $H_2O$), $NaHCO_3$ 5% $H_2O$. Sécher sur $MgSO_4$, filtrer et évaporer le solvant. Le produit est solubilisé dans le minimum de mélange chloroforme-MeOH 97,5—2,5 et déposé en tête d'une colonne (L: 24 cm ϕ 2,5) de gel de silice Merck $H^R$ type 60 montée dans le mélange. Eluer avec le même mélange et fractionner.

Nous obtenons un lot produit pur (rendement: 1 g) et deux lots de produit à recycler dans les mêmes conditions, d'où un rendement global:

Rendement: 67%; Fc: 95—8°C.

4. H-Sta-Ala-Sta-OMe, TFA

1 g de Boc-Ala-Sta-OMe est solubilisé dans 5 ml de chlorure de méthylène. Ajouter 10 ml de TFA à température ambiante et laisser ainsi 30 min. Evaporer les solvants sous trompe à eau. Reprendre l'huile résiduelle dans l'éther. Essorer le solide obtenu; rincer à l'éther; sécher.

Rendement: 850 mg (85%); Fc: 148—151°C.

5. Boc-Phe-Sta-Ala-Sta-OMe

470 g de H-Sta-Ala-Sta-OMe, TFA sont recouverts de 50 ml de chlorure de méthylène. Ajouter 102 mg de NEM et 382 mg de Boc-Phe-ONSu. Après une heure, le produit ayant du mal à se solubiliser, ajouter 10 ml de DMF; vérifier que le pH est à 6—7 au papier pH, sinon ajuster par N éthyl morpholine; agiter 20 h à température ambiante, puis évaporer les solvants à fond. Dissoudre le résidu dans le chlorure de méthylène et laver cette phase organique successivement par $KHSO_4$—$K_2SO_4$, eau, $NaHCO_3$, eau. Sécher sur $Na_2SO_4$; filtrer et évaporer le solvant.

Reprendre le résidu dans l'éther, essorer et sécher.

Rendement: 410 mg (70%).

6. H-Phe-Sta-Ala-Sta-OMe, TFA

400 g de Boc-Phe-Sta-Ala-Sta-OMe sont recouverts de 5 ml de TFA. Laissé 20 min à température ambiante, le solide s'est rapidement solubilisé. Evaporer le solvant sous vide. Reprendre l'huile résiduelle dans l'éther et agiter. Le solide obtenu est essoré, lavé à l'éther et séché.

Rendement: 400 mg (98%).

7. Z-Phe-Phe-Sta-Ala-Sta-OMe (SR 41320)

150 mg de H-Phe-Sta-Ala-Sta-OMe, TFA sont solublisés dans 10 ml de DMF. Ajouter 25 mg de NEM, puis 105 mg de Z-Phe-ONSu. Ajuster le pH à 6—7 au papier pH, par NEM si nécessaire, puis agiter 5 h à température ambiante. Evaporer le solvant sous haut vide, reprendre le résidu dans l'eau et le solide

obtenu est essoré, lavé à l'eau, séché, puis lavé à l'éther. Dissoudre dans méthanol-chloroforme 1/99 et introduire sur une colonne (L: 35 cm, ϕ 1 cm) de gel de silice 60 Merck[R] (70—230 Mesh) dans le même mélange. Eluer avec le même mélange 150 ml, puis avec méthanol-chloroforme 2/98:100 ml et méthanol-chloroforme 3/97:100 ml. Faire une CCM et évaporer les fractions pures. Reprendre dans l'éther et évaporer.

Nous obtenons une poudre blanche.

Rendement: 130 mg (69%); Fc: 188—191°C.

Exemple 2

Z-Phe-Phe-Sta-Ala-Sta-OH (SR 41225)

110 mg de Z-Phe-Phe-Sta-Ala-Sta-OMe (exemple 1) sont solubilisés dans 5 ml de DMF. Ajouter 2 ml d'eau. A température ambiante, ajouter ensuite 0,2 ml soude normale; agiter 30 min à température ambiante, puis ajouter 0,2 ml de ClH N; le pH est redescendu de 12,5 à 6,5. Evaporer les solvants, reprendre le résidu dans l'eau, essorer le solide, laver à l'eau, sécher.

Rendement: 70 mg (64%).

Analyse d'acides aminés: Ala: 1,03 (1)
Phe: 2,05 (2)
Sta: 1,92 (2)

Exemple 3

Z-Phe-(D)Phe-Sta-Ala-Sta-OMe (SR 41330)

1. Boc-(D)Phe-Sta-Ala-Sta-OMe

470 mg de H-Sta-Ala-Sta-OMe, TFA (exemple 1—4) sont recouverts de 50 ml de dioxane. Ajouter 102 mg de NEM, puis 382 mg de Boc(D)Phe-ONSu. Agiter à température ambiante. Après 24 heures, évaporer le solvant, reprendre le résidu dans le chlorure de méthylène et laver celui-ci successivement avec $KHSO_4$—$K_2SO_4$, eau, $NaHCO_3$, eau. Sécher sur $Na_2SO_4$ et évaporer le solvant. Reprendre dans l'éther et évaporer. De nouveau, reprendre dans l'éther et ajouter du pentane, le solide obtenu est essoré, dissous dans le chloroforme, et déposé en tête d'une colonne de gel de silice 60 Merck[R] (70—230 Mesh) dans le chloroforme (L: 10 cm, ϕ:3 cm). Eluer avec le chloroforme puis méthanol-chloroforme 1/99; fractionner. Evaporer les fractions pures. Reprendre dans éther-pentane, essorer, sécher.

Rendement: 200 mg (37%).

2. H-(D)Phe-Sta-Ala-Sta-OMe, TFA

200 mg de Boc(D)Phe-Sta-Ala-Sta-OMe sont recouverts dé 2 ml de TFA. Après 30 min à température ambiante, évaporer le solvant, reprendre dans l'éther et le solide blanc obtenu est essoré, séche.

Rendement: 190 mg (93%).

3. Z-Phe-(D)-Phe-Sta-Ala-Sta-OMe (SR 41330)

190 mg de H-(D)Phe-Sta-Ala-Sta-OMe, TFA sont solubilisés dans 25 ml de dioxane contenant 64 mg de NEM. Ajouter 133 mg de Z-Phe-ONSu et 45 mg de HOBt, agiter à température ambiante. Ajuster le pH à 6—7 par NEM si nécessaire. Après 24 heures, évaporer le solvant, reprendre le résidu dans l'eau, triturer, laisser reposer 30 minutes, essorer, laver à l'éther, sécher.

Rendement: 190 mg

Analyse d'acides aminés: Ala: 0,97 (1)
Phe: 2,00 (2)
Sta: 2,14 (2).

Spectre de RMN:

| δ | Aspects | Intégration | Attribution | |
|---|---|---|---|---|
| 0,75 | m | 12 H | $^{6}C(CH_3)_2$ | Sta |
| 1,09—1,60 | m | 9 H | $^{5}CH_2$ $^{6}CH$ | Sta |
| | | | $CH_3$ | Ala |
| 2,08—2,37 | m | 4 H | $^{2}CH_2$ | Sta |
| 2,43—3,05 | m | 4 H | $^{3}CH_2$ | Phe |
| 3,49 | s | 3 H | $CH_3$ | ester |
| 3,79 | m | 4 H | $^{4}CH—^{3}CH$ | Sta |
| 4,16 | m | 2 H | $^{2}CH$ (D) | Phe, Phe, Ala |
| 4,53 | m | 1 H | | |
| 4,82 | d | 4 H | OH | Sta |
| 4,85 | d | | $CH_2$ | Z |
| 4,89 | d | | OH | Sta |
| 7,04—7,26 | m | 17 H | aromatiques | |
| 7,31 | d | | NH | |
| 7,36 | d | | NH | |
| 7,52 | d | 1 H | NH | |
| 7,84 | d | 1 H | NH | |
| 8,27 | d | 1 H | NH | |

Exemple 4

Boc-Phe-Phe-Sta-Ala-Sta-OMe (SR 41331)

230 mg de H-Phe-Sta-Ala-Sta-OMe, TFA (Exemple 1—6) sont solublisés dans 25 ml de dioxane contenant 80 mg de NEM. Ajouter 145 mg de Boc-Phe-ONSu et 54 mg de HOBt, agiter à température ambiante et ajuster à pH 6—7 par NEM, si nécessaire. Après 24 heures, évaporer le solvant, reprendre le résidu dans l'eau, triturer. Laisser reposer 30 minutes, filtrer, laver à l'éau, puis à l'éther; sécher.

Rendement: 180 mg (65%).

Analyse d'acides aminés: Ala: 1,04 (1)
Phe: 2,00 (2)
Sta: 1,83 (2).

Spectre de RMN

| δ | Aspects | Intégration | Attribution | |
|---|---|---|---|---|
| 0,77 | m | 12 H | $^6C(CH_3)_2$ | Sta |
| 1,02—1,58 | m | 18 H | $^5CH_2$—$^6CH$ | Sta |
| | | | $CH_3$ | Ala |
| | | | Boc | Phe |
| 1,90—2,38 | m | 4 H | $^2CH_2$ | Sta |
| 2,46—3,23 | m | 4 H | $^3CH_2$ | Phe |
| 3,49 | s | 3 H | $CH_3$ | ester |
| 3,74 | m | 4 H | $^4CH$—$^3CH$ | Sta |
| 4,05 | m | 1 H | | |
| 4,20 | m | 1 H | $^2CH$ | Phe, Phe, Ala |
| 4,52 | m | 1 H | | |
| 4,78 | d(J=6 Hz) | 1 H | OH | Sta |
| 4,93 | d( „ ) | 1 H | OH | Sta |
| 6,82 | d | 1 H | NH | |
| 7,04 | m | 10 H | aromatiques | |
| 7,34 | d | 1 H | NH | |
| 7,52 | d | 1 H | NH | |
| 7,77 | d | 1 H | NH | |
| 7,93 | d | 1 H | NH | |

Exemple 5

Boc-Trp-Trp-Sta-Ala-Sta-OMe (SR 41376)

1. Boc-Trp-Sta-Ala-Sta-OMe

470 mg de H-Sta-Ala-Sta-OMe, TFA (exemples 1—4) sont solubilisés dans 50 ml de dioxane contenant 102 mg de NEM. Ajouter 380 mg de Boc-Trp-ONp et 120 mg de HOBt. Agiter à température ambiante, ajuster le pH à 6—7 par NEM; si nécessaire. Après 48 h, évaporer le solvant sous vide, reprendre le résidu dans le chloroforme et disposer en tête d'une colonne de gel de silice 60 Merck[R] (70—230 Mesh) dans le chloroforme (L: 15 cm, ϕ: 3 cm). Eluer avec: 300 ml de chloroforme, 200 ml de méthanol-chloroforme 1/99, 200 ml de méthanol-chloroforme 2/98, 200 ml de méthanol-chloroforme 3/97, 500 ml de méthanol-chloroforme 5/95. Fractionner en fractions de 25 ml, faire le suivi en CCM, évaporer les fractions pures, reprendre dans le pentane avec un peu d'éther, essorer, sécher.

Rendement: 320 mg (56%).

2. H-Trp-Sta-Ala-Sta-OMe, TFA

200 mg de Boc-Trp-Sta-Ala-Sta-OMe, TFA sont recouverts de 5 ml de chlorure de méthylène auxquels on ajoute 4 gouttes d'éthane dithiol et 2 gouttes d'anisol, puis 2 ml de TFA. Laisser à température ambiante, évaporer les solvants et reprendre le résidu dans l'éther. Essorer le solide, laver abondamment à l'éther, sécher.

Rendement: 170 mg.

3. Boc-Trp-Trp-Sta-Ala-Sta-OMe (SR 41376)

170 mg de H-Trp-Sta-Ala-Sta-OMe, TFA sont recouverts de 30 ml de dioxane contenant 60 mg de NEM.

Ajouter 117 mg de Boc-Trp-ONp et 38 mg de HOBt. Agiter à température ambiante, ajuster le pH à 6—7 par NEM, si nécessaire. Après 24 heures, évaporer le solvant et reprendre le résidu dans l'eau; laisser au repos. Essorer le solide obtenu et dissoudre dans le chlorure de méthylène et laver la solution organique avec $KHSO_4$—$K_2SO_4$, eau, $NaHCO_3O$, eau.

Sécher sur $Na_2SO_4$; évaporer le solvant. Dissoudre le produit dans 3 ml de chloroforme et déposer en tête d'une colonne (L: 30 cm, φ: 2 cm) de gel de silice 60 Merck[R] (70—230 Mesh) dans le chloroforme. Eluer avec chloroforme 100 ml, méthanol-chloroforme 1/99 100 ml et méthanol-chloroforme 5/95 jusqu'à sortie du produit. Fractionner, évaporer les fractions pures. Reprendre le résidu dans l'éther, essorer, sécher.

Rendement: 90 mg (44%)

Analyse d'acides aminés: Ala: 0,98 (1)
Trp: 2,08 (2)
Sta: 1,97 (2).

Spectre de RMN

| δ | Aspects | Intégration | Attribution | |
|---|---|---|---|---|
| 0,69—0,85 | m | 12 H | $^6C(CH_3)_2$ | Sta |
| 0,93—1,58 | m | 18 H | $CH_3$ | Ala |
| | | | $^5CH_2$—$^6CH$ | Sta |
| | | | $(CH_3)_3C$ | Boc |
| 1,90—2,38 | m | 4 H | $^2CH_2$ | Sta |
| 2,69—3,15 | m | 4 H | $^3CH_2$ | Trp |
| 3,50 | s | 3 H | $CH_3$ | ester |
| 3,71—3,89 | m | 4 H | $^4CH$—$^3CH$ | Sta |
| 4,00—4,27 | m | 2 H | $^2CH$ | Ala |
| 4,53 | m | 1 H | $2\times{}^2CH$ | Trp |
| 4,75 | d | 2 H | 2×OH | Sta |
| 4,94 | d | | | |
| 6,72—7,92 | m | 15 H | aromatiques<br>5×NH | |

Exemple 6

Boc-(D)Phe-Val-Sta-Ala-Sta-$OCH_3$ (SR 41377)

1. Boc-Val-Sta-Ala-Sta-OMe

450 mg de H-Sta-Ala-Sta-OMe, TFA (exemple 1—4) sont recouverts de 50 ml de dioxane contenant 211 mg de NEM. Ajouter 314 mg de Boc-Val-ONSu et 135 mg de HOBt. Agiter à TA, ajuster le pH à 6—7 par NEM, si nécessaire. Après 24 h, évaporer le solvant, reprendre le résidu dans l'eau, essorer le solide obtenu après 3 h, sécher. Dissoudre le produit brut dans le chloroforme, le déposer en tête d'une colonne (L: 18 cm, φ: 3 cm) de gel de silice 60 Merck[R] (70—230 Mesh) dans le même solvant. Eluer en faisant un gradient de méthanol-chloroforme jusqu'à 7/93 (vol/vol) par fractions de 200 ml de solvant, fractionner. Evaporer les fractions pures.

Rendement: 200 mg (38%).

2. H-Val-Sta-Ala-Sta-OMe

180 mg de Boc-Val-Sta-Ala-Sta-OMe sont recouverts de 3 ml de TFA. Après 30 min à température ambiante, évaporer le solvant à froid, reprendre le résidu dans l'éther, essorer le solide blanc obtenu, laver à l'éther, sécher.

Rendement: 180 mg (100%).

3. Boc-(D)Phe-Val-Sta-Ala-Sta-OMe

150 mg de H-Val-Sta-Ala-Sta-OMe, TFA sont recouverts de 35 ml de dioxane contenant 90 mg de NEM.

Ajouter 101 mg de Boc-(D)Phe-ONSu et 38 mg de HOBt. Ajouter 3 ml de DMF, ajuster le pH à 6—7 par NEM. Après 20 heures, évaporer les solvants, reprendre le résidu dans l'eau et le solide obtenu est essoré, lavé à l'eau, puis à l'éther; sécher.

Rendement: 150 mg (85%).

Analyse d'acides aminés: Ala: 1,00 (1)
Val: 1,08 (1)
Phe: 0,97 (1)
Sta: 1,96 (2).

Spectre de RMN

| δ | Aspects | Intégration | Attribution | |
|---|---|---|---|---|
| 0,67—0,83 | m | 18 H | $^6C(CH_3)_2$ | Sta |
| | | | $^3C(CH_3)_2$ | Val |
| | | | $^5CH_2—^6CH$ | Sta |
| 1,09—1,58 | m | 18 H | $(CH_3)_3C$ | Boc |
| | | | $CH_3$ | Ala |
| 1,92 | m | 1 H | $^3CH$ | Val |
| 2,00—2,35 | m | 4 H | $^2CH_2$ | Sta |
| 2,50—2,94 | m | 2 H | $^3CH_2$ | (D)Phe |
| 3,50 | s | 3 H | $CH_3$ | ester |
| 3,78 | m | 4 H | $^4CH—^3CH$ | Sta |
| 4,08—4,26 | m | 3 H | $^2CH$ | (D) Phe, Val, Ala |
| 4,75 | d | 1 H | OH | Sta |
| 4,91 | d | 1 H | OH | Sta |
| 6,90 | d | 1 H | NH | |
| 7,06—7,33 | m | 6 H | aromat.+1 NH | |
| 7,50 | d | 1 H | NH | |
| 7,75 | d | 1 H | NH | |
| 7,82 | d | 1 H | NH | |

Exemple 7

Boc-Trp-(D)Trp-Sta-Ala-Sta-OMe (SR 41394)

1. Boc-(D)Trp-Sta-Ala-Sta-OMe

470 mg de H-Sta-Ala-Sta-OMe, TFA (Exemple 1—4) sont recouverts de 50 ml de dioxane contenant 195 mg de NEM; puis ajouter 380 mg de Boc-(D)Trp-ONp et 120 mg de HOBt. Agiter à TA, ajuster le pH à 6—7 au papier pH par NEM, si nécessaire. Après 6 jours évaporer le solvant, reprendre dans le chlorure de méthylène et laver la solution organique successivement par $KHSO_4—K_2SO_4$, par l'eau, $NaHCO_3$, par l'eau. Sécher sur $Na_2SO_4$. Evaporer le solvant à sec, dissoudre le produit dans le chloroforme et déposer en tête d'une colonne (L: 27 cm, ϕ: 2 cm) de gel de silice Merck[R] (70—230 Mesh) dans le chloroforme. Eluer en faisant un gradient méthanol-chloroforme jusqu'à 5/95. Fractionner, évaporer les fractions pures.

Rendement: 330 mg (58%).

2. H-(D)Trp-Sta-Ala-Sta-OMe, TFA

220 mg de Boc-(D)Trp-Sta-Ala-Sta-OMe sont recouverts de 10 ml de chlorure de méthylène. Ajouter 8

gouttes d'éthane dithiol et 2 ml de TFA. Après 30 min à TA, évaporer les solvants. Reprendre le résidu dans l'éther, ajouter un peu de pentane, essorer, laver à l'éther; sécher.

Rendement: 210 mg (95%).

3. Boc-Trp-(D)Trp-Sta-Ala-Sta-OMe (SR 41394)

120 mg de H(D)Trp-Sta-Ala-Sta-OMe, TFA sont solubilisés dans 10 ml de dioxane contenant 41 mg de NEM. Ajouter 85 mg de Boc-Trp-ONp et 27 mg de HOBt. Agiter à TA et vérifier que le pH est à 6—7 au papier pH, sinon ajuster par NEM. Après 48 heures, évaporer le solvant, reprendre le résidu dans le chlorure de méthylène et laver la solution organique successivement par $KHSO_4$—$K_2SO_4$, par l'eau, $NaHCO_3$, l'eau. Sécher sur $Na_2SO_4$, évaporer le solvant. Dissoudre dans l'acétate d'éthyle et passer sur un gel de silice 60 Merck[R] (70—230 Mesh) (H: 30 cm, φ: 3 cm) et éluer avec l'acétate d'éthyle, puis progressivement avec le méthanol. Fractionner, évaporer les fractions pures, reprendre le résidu dans l'éther, essorer; sécher.

Rendement: 120 mg (85%)

Analyse d'acides aminés: Ala: 1,05 (1)
Sta: 1,91 (2)
Trp: 2,04 (2).

Spectre de RMN

| δ | Aspects | Intégration | Attribution | |
|---|---|---|---|---|
| 0,59—0,83 | m | 12 H | $^6C(CH_3)_2$ | Sta |
| 0,91—1,57 | m | 18 H | $CH_3$ | Ala |
| | | | $^5CH_2$—$^6CH$ | Sta |
| | | | $(CH_3)_3C$ | Boc |
| 2,03—2,38 | m | 4 H | $^2CH_2$ | Sta |
| 2,56—3,23 | m | 4 H | $^3CH_2$ | Trp |
| 3,50 | s | 3 H | $CH_3$ | ester |
| 3,70—3,89 | m | 4 H | $^4CH$—$^3CH$ | Sta |
| 4,05—4,24 | m | 2 H | $^2CH$ | Ala |
| 4,41—4,54 | m | 1 H | 2×$^2CH$ | Trp |
| 4,75 | d | 2 H | 2×OH | Sta |
| 4,90 | d | | | |
| 6,58 | d | 1 H | NH | |
| 6,78—7,67 | m | 12 H | aromatiques | |
| | | | 2×NH | |
| 7,81 | d | 1 H | NH | |
| 8,03 | d | 1 H | NH | |
| 10,69 | s | 1 H | NH | indole |
| 10,74 | s | 1 H | NH | indole |

Exemple 8

Boc-Phe-His-Sta-Ala-Sta-OMe (SR 41395)

1. Boc-His(Boc)-Sta-OMe

0,8 g (0,0015 M) de Boc-His(Boc)-OH, DCHA sont solubilisés dans 80 ml de chlorure de méthylène; ajouter 306 mg de H-Sta-OMe, TFA et agiter jusqu'à dissolution, puis ajouter 0,8 g de Bop et 233 mg de

DIPEA. Contrôler le pH au cours du temps, et ajuster à 6—7 par DIPEA, si nécessaire. Après 24 heures, évaporer le solvant. Dissoudre le produit dans l'acétate d'éthyle et déposer en tête d'une colonne (L: 60 cm, ϕ: 3 cm) de gel de silice 60 Merck[R] (70—230 Mesh) dans l'acétate d'éthyle. Eluer avec l'acétate d'éthyle, fractionner. Evaporer les fractions pures. Reprendre dans hexane, évaporer l'hexane et le solide obtenu est séché.

Rendement: 610 mg (77%).

2. Boc-Phe-His-Sta-OMe

350 mg de Boc-His(Boc)-Sta-OMe sont recouverts de 5 ml de TFA à température ambiante. Après 30 minutes, évaporer à fond celui-ci. Dissoudre dans 50 ml de dioxane, puis ajouter 237 mg de NEM et vérifier que le pH est à 6—7 sinon ajuster. Ajouter 89 mg de HOBt et 260 mg de Boc-Phe-ONSu. Ajuster le pH à 6—7 par NEM, si nécessaire. Agiter 24 heures à TA, évaporer le solvant. Dissoudre dans acétate d'éthyle-méthanol 95/5 et chromatographier sur une colonne (L: 40 cm, ϕ: 2 cm) de gel de silice 60 Merck[R] (70—230 Mesh) dans le même solvant. Eluer avec 300 ml du même mélange solvant, puis avec acétate d'éthyle-méthanol 90/10; fractionner et évaporer les fractions pures.

Rendement: 130 mg (34%).

3. Boc-Phe-His-Sta-OH

450 mg de Boc-Phe-His-Sta-OMe sont solubilisés dans 20 ml de méthanol. Ajouter 5 ml d'eau, puis 200 mg de baryte en poudre. Après 45 minutes, ajouter à nouveau 100 mg de baryte. Après 1 h 30, la réaction est arrêtée; faire barboter du gaz carbonique durant 30 minutes et filtrer sur célite; laver celle-ci avec du méthanol. Evaporer les solvants; reprendre le résidu dans l'éther, essorer, sécher.

Rendement: 400 mg.

4. Boc-Phe-His-Sta-Ala-Sta-OMe

180 mg de Boc-Ala-Sta-OMe sont recouverts de 5 ml de TFA. Après 30 minutes à température ambiante, évaporer le solvant, ajouter de l'éther, et de nouveau évaporer. Dissoudre l'huile dans 10 ml de DMF et ajouter DIPEA jusqu'à l'obtention de pH 6—7 au papier pH. Ajouter ensuite 230 mg de Boc-Phe-His-Sta-OH dans 10 ml de DMF contenant 53 mg de DIPEA, puis une solution de 268 mg de Bop dans 10 ml de DMF contenant 76 mg de DIPEA; agiter à TA, ajuster le pH à 6—7 si nécessaire par DIPEA. Après 24 heures, évaporer le solvant à fond sous haut vide au bain-marie à 40°. Reprendre le résidu dans l'eau, extraire avec du chlorure de méthylène et laver la solution organique avec de l'eau, $NaHCO_3$, de l'eau. Sécher sur $Na_2SO_4$, puis évaporer le solvant, dissoudre le résidu dans méthanol-chloroforme 2/98 et chromatographier sur une colonne (L: 35 cm, ϕ: 2 cm) de gel de silice 60 Merck[R] (70—230 Mesh) dans le même mélange solvant. Eluer avec un gradient méthanol-CH jusqu'à 10/90. Fractionner, évaporer les fractions pures. Reprendre le résidu dans éther et essorer; sécher.

Rendement: 150 mg (45%)

Analyse d'acides aminés: Ala: 1,01 (1)
Phe: 0,98 (1)
His: 0,99 (1).

Spectre de RMN

| δ | Aspects | Intégration | Attribution | |
|---|---|---|---|---|
| 0,67—0,86 | m | 12 H | $^6C(CH_3)_2$ | Sta |
| | | | $CH_3$ | Ala |
| 0,86—1,57 | m | 18 H | $(CH_3)_3C$ | Boc |
| | | | $^5CH_2—^6CH$ | Sta |
| 1,97—2,38 | m | 4 H | $^2CH_2$ | Sta |
| 2,61—2,96 | m | 4 H | $^3CH_2$ | Phe, His |
| 3,50 | s | 3 H | $CH_3$ | ester |
| 3,65—3,87 | m | 4 H | $^4CH—^3CH$ | Sta |
| 4,03—4,26 | m | 2 H | $^2CH$ | Phe, Ala |
| 4,42 | m | 1 H | $^2CH$ | His |
| 4,93 | m | 2 H | OH | Sta |
| 6,77 | s | 1 H | $^{4'}CH$ | His |
| 6,98 | d | 1 H | NH | |
| 7,08—7,27 | m | 5 H | aromatiques | |
| 7,33 | d | 2 H | NH | |
| 7,38 | d | | NH | |
| 7,52 | s | 1 H | $^{2'}CH$ | His |
| 7,96 | d | 1 H | NH | |
| 8,14 | d | 1 H | NH | |

Exemple 9

Z-Phe-Val-Sta-Ala-Sta-OMe (SR 41405)

100 mg de H-Val-Sta-Ala-Sta-OMe, TFA (exemple 6—2) sont recouverts de 10 ml de dioxane contenant 40 mg de NEM. Ajouter 75 mg de Z-Phe-ONSu et 25,5 mg de HOBt. Agiter à température ambiante, ajuster le pH à 6—7 par NEM, si nécessaire. Après 24 heures, évaporer le solvant, reprendre le résidu dans l'eau. Essorer le solide, laver à l'éther; sécher.

Rendement: 105 mg (81%)

Analyse d'acides aminés: Ala: 1,00 (1)
Val: 0,99 (1)
Phe: 1,03 (1)
Sta: 1,98 (2).

Spectre de RMN

| δ | Aspects | Intégration | Attribution | |
|---|---|---|---|---|
| 0,69—0,89 | m | 18 H | $^{6}C(CH_3)_2$ | Sta |
| | | | $^{3}C(CH_3)_2$ | Val |
| 1,07—1,57 | m | 9 H | $^{5}CH_2$—$^{6}CH$ | Sta |
| | | | $CH_3$ | Ala |
| 1,81—2,37 | m | 5 H | $^{3}CH$ | Val |
| | | | $^{2}CH_2$ | Sta |
| 2,58—2,98 | m | 2 H | $^{3}CH_2$ | Phe |
| 3,50 | s | 3 H | $CH_3$ | ester |
| 3,78 | m | 4 H | $^{4}CH$—$^{3}CH$ | Sta |
| 4,06—4,34 | m | 3 H | $^{2}CH$ | Phe, Val, Ala |
| 4,78 | d | 4 H | OH | Sta |
| 4,86 | s | | $CH_2$ | Z |
| 4,90 | d | | OH | Sta |
| 7,00—7,34 | m | 11 H | aromatiques | |
| | | | 1 NH | |
| 7,42 | m | 2 H | 2 NH | |
| 7,79 | d | 1 H | NH | |
| 7,87 | d | 1 H | NH | |

Exemple 10

Z-Phe-(D)Phe-Sta-Ala-Sta-OH (SR 41406)

110 mg de Z-Phe-(D)Phe-Sta-Ala-Sta-OMe (exemple 3) sont solubilisés dans 5 ml de DMF. Ajouter 2 ml d'eau, puis à TA, ajouter 0,2 ml de soude normale (1,5 équivalent). Agiter 30 minutes à température ambiante, puis ajouter 0,2 ml d'acide chlorhydrique normal (le pH est alors à 6 au papier pH). Evaporer les solvants sous haut vide au bain-marie à 40°. Reprendre le résidu dans l'eau, triturer et essorer le solide; sécher.

Rendement: 90 mg (83%)

Analyse d'acides aminés: Ala: 0,91 (1)
Phe: 2,00 (2)
Sta: 2,03 (2).

Exemple 11

Z-Phe-(D)Trp-Sta-Ala-Sta-OMe (SR41407)

80 mg de H-(D)Trp-Sta-Ala-Sta-OMe (exemple 7) sont recouverts de 10 ml de dioxane contenant 27 mg de NEM, puis ajouter 51 mg de 3-Phe-ONSu et 17 mg de HOBt. Ajuster le pH à 6—7 au papier pH, si nécessaire et agiter à TA; après 24 heures, évaporer le solvant sous vide, reprendre le résidu dans l'eau, après une heure, essorer le solide, puis le dissoudre dans le chlorure de méthylène et laver la solution organique successivement avec $KHSO_4$—$K_2SO_4$, eau, $NaHCO_3$, eau. Sécher sur $Na_2SO_4$ et évaporer le solvant. Dissoudre le produit dans méthanol-chloroforme 2/98 et le déposer en tête d'une colonne (L: 40 cm, ϕ: 2 cm) de gel de silice 60 Merck[R] (70—230 Mesh) dans le même mélange de solvant. Eluer avec 200 ml de méthanol-chloroforme 2/98 et 400 ml de méthanol-chloroforme 5/95. Fractionner, faire une CCM et évaporer les fractions pures. Reprendre dans l'éther, essorer; sécher.

Rendement: 50 mg (51%)
Analyse d'acides aminés: Ala: 1,01 (1)
Phe: 1,01 (1)
Sta: 1,99 (2)
Trp: 1 (1).

Spectre de RMN

| δ | Aspects | Intégration | Attribution | |
|---|---|---|---|---|
| 0,64—0,86 | m | 12 H | $^6C(CH_3)_2$ | Sta |
| 1,00—1,60 | m | 9 H | { $CH_3$ | Ala |
| | | | $^5CH_2—^6CH$ | Sta |
| 2,10—2,42 | m | 4 H | $^2CH_2$ | Sta |
| 2,48—3,20 | m | 4 H | $^3CH_2$ | Phe, Trp |
| 3,50 | s | 3 H | $CH_3$ | ester |
| 3,71—3,89 | m | 4 H | $^4CH—^3CH$ | Sta |
| 4,13—4,26 | m | 2 H | $^2CH$ | Phe, Ala |
| 4,51 | m | 1 H | $^2CH$ | Trp |
| 4,75—4,95 | m | 4 H | { 2 OH | Sta |
| | | | $CH_2$ | Z |
| 6,85—7,49 | m | 17 H | { aromatiques | |
| | | | 2 NH | |
| 7,60 | d | 1 H | NH | |
| 7,84 | d | 1 H | NH | |
| 8,24 | d | 1 H | NH | |

Exemple 12

Z-Phe-Trp-Sta-Ala-Sta-OMe (SR 41416)

80 mg de H-Trp-Sta-Ala-Sta-OMe, TFA (exemple 5) sont solubilisés dans 20 ml de dioxane contenant 27 mg de NEM puis ajouter 51 mg de Z-Phe-ONSu et 17 mg de HOBt. Agiter à TA, ajuster le pH à 6—7 par NEM, si nécessaire. Après 48 heures, évaporer le solvant, reprendre le résidu dans l'eau et le solide est essoré, Laver successivement avec $KHSO_4—K_2S$ puis l'eau, puis $CO_3HNa$ puis l'eau. Dissoudre dans acétate d'éthyle, sécher sur $MgSO_4$, puis évaporer à sec. Triturer dans l'éther, essorer, sécher.

Rendement: 90 mg (92%)
Analyse d'acides aminés: Ala: 0,97 (1)
Phe: 0,99 (1)
Sta: 2,00 (2)
Trp: 1,04 (1).

Spectre de RMN

| δ | Aspects | Intégration | Attribution | |
|---|---|---|---|---|
| 0,69—0,85 | m | 12 H | $^6C(CH_3)_2$ | Sta |
| 1,09—1,58 | m | 9 H | { $CH_3$ | Ala |
| | | | { $^5CH_2—^6CH$ | Sta |
| 1,09—2,4 | m | 4 H | $^2CH_2$ | Sta |
| 2,53—3,20 | m | 4 H | $^3CH_2$ | Phe, Trp |
| 3,50 | s | 3 H | $CH_3$ | ester |
| 3,79 | m | 4 H | $^4CH—^3CH$ | Sta |
| 4,11—4,27 | m | 2 H | $^2CH$ | Phe, Ala |
| 4,51 | m | 1 H | $^2CH$ | Trp |
| 4,75—5,00 | m | 4 H | { 2 OH | Sta |
| | | | { $CH_2$ | Z |
| 6,86—7,50 | m | 17 H | { aromatiques | |
| | | | { 2 NH | |
| 7,57 | d | 1 H | NH | |
| 7,79 | d | 1 H | NH | |
| 8,19 | d | 1 H | NH | |
| 10,69 | s | 1 H | NH | indole |

Exemple 13

Z-Tyr-Val-Sta-Ala-Sta-OMe (SR 41476)

100 mg de H-Val-Sta-Ala-Sta-OMe, TFA (exemple 6—2) sont recouverts de 10 ml de dioxane contenant 45 mg de NEM. Ajouter 82,5 mg de Z-Tyr-ONSu et 27,5 mg de HOBt. Ajuster le pH à 6—7 par NEM, si nécessaire. Agiter 48 heures à température ambiante. Evaporer le solvant à froid, reprendre le résidu dans l'eau et le solide blanc obtenu est erroré, lavé, à l'eau et séché. Laver à l'éther et sécher.

Rendement: 110 mg (85%)

Analyse d'acides aminés: Ala: 1,02 (1)
Val: 0,99 (1)
Tyr: 0,99 (1)
Sta: 2,01 (2).

Spectre de RMN

| δ | Aspects | Intégration | Attribution | |
|---|---|---|---|---|
| 0,69—0,88 | m | 18 H | $^{6}C(CH_3)_2$ | Sta |
| | | | $^{3}C(CH_3)_2$ | Val |
| 1,09—1,58 | m | 9 H | $CH_3$ | Ala |
| | | | $^{5}CH_2—^{6}CH$ | Sta |
| 1,82—2,00 | m | 1 H | $^{3}CH$ | Val |
| 2,00—2,40 | m | 4 H | $^{2}CH_2$ | Sta |
| 2,50—2,88 | m | 2 H | $^{3}CH_2$ | Tyr |
| 3,50 | s | 3 H | $CH_3$ | ester |
| 3,70—3,86 | m | 4 H | $^{4}CH—^{3}CH$ | Sta |
| 4,06—4,27 | m | 3 H | $^{2}CH$ | Tyr, Val, Ala |
| 4,80 | d | 4 H | OH | Sta |
| 4,88 | s | | $CH_2$ | Z |
| 4,91 | d | | OH | Sta |
| 6,58 | d | 2 H | 3',5' CH | arom. Tyr |
| 7,00 | d | 2 H | 2',6' CH | arom. Tyr |
| 7,09—7,50 | m | 8 H | aromatiques Z | |
| | | | 3×NH | |
| 7,77—7,90 | m | 2 H | 2×NH | |

Exemple 14

Z-Phe-ILe-Sta-Ala-Sta-OMe (SR 41477)

1. Boc-Ile-Sta-Ala-Sta-OMe

265 mg de H-Sta-Ala-Sta-OMe, TFA (exemple 1—4) sont recouverts de 30 ml de dioxane contenant 206 mg de DIPEA. Ajouter 125 mg de Boc-Ile-OH et 270 mg de Bop. Ajuster le pH à 6—7 par DIPEA, si nécessaire, et agiter 48 heures à TA. Evaporer le solvant sous vide, reprendre le résidu dans l'éther, essorer le solide. Déposer le produit en solution dans méthanol-chloroforme 2,5/97,5 en tête d'une colonne (L: 30 cm, ϕ: 2 cm) de gel de silice 60 Merck$^{R}$ (70—230 Mesh) dans le même solvant. Eluer avec le même mélange solvant (250 ml) puis avec 200 ml de méthanol-chloroforme 5/95 et 200 ml de méthanol-chloroforme 7,5/92,5. Fractionner, évaporer les bonnes fractions, reprendre dans l'éther, essorer, sécher.

Rendement: 180 mg (56%).

2. H-Ile-Sta-Ala-Sta-OMe, TFA

150 mg de Boc-Ile-Sta-Ala-Sta-OMe sont recouverts de 2 ml de TFA. Après 30 minutes, évaporer le solvant, reprendre le résidu dans l'éther et le solide blanc est essoré, lavé à l'éther et séché.

Rendement: 140 mg (93%).

3. Z-Phe-Ile-Sta-Ala-Sta-OMe (SR 41477)

126 mg de H-Ile-Sta-Ala-Sta-OMe, TFA sont recouverts de 20 ml de dioxane contenant 51 mg de NEM. Ajouter 95 mg de Z-Phe-ONSu et 32 mg de HOBt. Ajuster le pH à 6—7, si nécessaire, par NEM, et agiter 48 heures à TA, évaporer le solvant sous vide. Reprendre le résidu dans l'eau, triturer, essorer, sécher. Laver à l'éther, sécher.

Rendement: 140 mg (86%)
Analyse d'acides aminés: Ala: 0,96 (1)
Ile: 0,98 (1)
Phe: 1,03 (1)
Sta: 2,03 (2).

Spectre de RMN

| δ | Aspects | Intégration | Attribution | |
|---|---|---|---|---|
| 0,64—0,91 | m | 18 H | $^6C(CH_3)_2$ | Sta |
| | | | $^3CH_3$ $^4CH_3$ | Ile |
| | | | $CH_3$ | Ala |
| 0,91—1,81 | m | 12 H | $^5CH_2$—$^6CH$ | Sta |
| | | | $^4CH_2$—$^3CH$ | Ile |
| 2,00—2,39 | m | 4 H | $^2CH_2$ | Sta |
| 2,50—2,96 | m | 2 H | $^3CH_2$ | Phe |
| 3,50 | s | 3 H | $CH_3$ | ester |
| 3,79 | m | 4 H | $^4CH$—$^3CH$ | Sta |
| 4,08—4,35 | m | 3 H | $^2CH$ | Phe, Ile, Ala |
| 4,80 | d | | OH | Sta |
| 4,88 | s | 4 H | $CH_2$ | Z |
| 4,93 | d | | OH | Sta |
| 7,09—7,36 | m | 11 H | aromatiques+1NH | |
| 7,44 | 2 d | 2 H | 2 NH | |
| 7,79 | d | 1 H | NH | |
| 7,94 | d | 1 H | NH | |

Exemple 15

Z-PHe-(D)Val-Sta-Ala-Sta-OMe (SR 41478)

1. Boc-(D)Val-Sta-Ala-Sta-OMe

531 mg de H-Sta-Ala-Sta-OMe, TFA (exemple 1—4) sont recouverts de 50 mg de dioxane contenant 253 mg de NEM; puis ajouter 380 mg de Boc-(D)Val-ONSu et 135 mg de HOBt. Ajuster le pH à 6—7 par NEM, si nécessiare, et agiter 3 jours à TA; évaporer le solvant sous vide, reprendre le résidu dans l'eau et extraire au chlorure de méthylène. Laver la phase organique à l'eau, sécher sur $Na_2SO_4$ et évaporer le solvant. Reprendre le résidu dans l'éther, laisser reposer. Le précipité blanc formé est essoré sous vide et séché.

Rendement: 250 mg (40%).

2. H-(D)Val-Sta-Ala-Sta-OMe, TFA

150 mg de Boc-(D)Val-Sta-Ala-Sta-OMe sont recouverts de 2 ml de TFA. Après 30 minutes à température ambiante, évaporer le solvant à fond. Reprendre le résidu dans l'éther et le solide obtenu est essoré sous vide, lavé à l'éther et séché.

Rendement: 150 mg (100%).

3. Z-Phe-(D)Val-Sta-Ala-Sta-OMe

Dans 25 ml de dioxane contenant 40 mg de NEM on introduit successivement 100 mg de H-(D)Val-Sta-Ala-Sta-OMe, TFA puis 75 mg de Z-Phe-ONSu et 25,5 mg de HOBt. Agiter à TA, 3 jours après avoir ajusté le pH à 6—7, si nécessaire. Evaporer le solvant sous vide, laver à l'eau, puis à l'éther; sécher.

Rendement: 80 mg (29%)
Analyse d'acides aminés: Ala: 0,97 (1)
Val: 1,03 (1)
Phe: 1,01 (1)
Sta: 1,98 (2).

Spectre de RMN

| δ | Aspects | Intégration | Attribution | |
|---|---|---|---|---|
| 0,62—0,85 | m | 18 H | $^6C(CH_3)_2$ | Sta |
| | | | $^3C(CH_3)_2$ | Val |
| 1,06—1,57 | m | 9 H | $^5CH_2—^6CH$ | Sta |
| | | | $CH_3$ | Ala |
| 1,92 | m | 1 H | $^3CH$ | Val |
| 2,06—2,35 | m | 4 H | $^2CH_2$ | Sta |
| 2,50—3,00 | m | 2 H | $^3CH_2$ | Phe |
| 3,51 | s | 3 H | $CH_3$ | ester |
| 3,77 | m | 4 H | $^4CH—^3CH$ | Sta |
| 4,10—4,38 | m | 3 H | $^2CH$ | Phe, (D)Val, Ala |
| 4,72—4,94 | m | 4 H | 2 OH | Sta |
| | | | $CH_2$ | Z |
| 7,04—7,92 | m | 15 H | aromatiques | |
| | | | 5×NH | |

Exemple 16
Z-Trp-Val-Sta-Ala-Sta-OMe (SR41485)

70 mg de H-Val-Sta-Ala-Sta-OMe, TFA (exemple 6), sont recouverts de 20 ml de dioxane contenant 30 mg de NEM. Ajouter à TA 67 mg de Z-Trp-ONp et 20 mg de HOBt. Ajuster le pH à 6—7 par NEM, si nécessaire, et agiter à TA; après 48 heures, évaporer le solvant sous vide, reprendre le résidu huileux dans l'eau, décanter l'eau et reprendre dans l'éther. Le solide obtenu est essoré, lavé à l'éther, séché.

Rendement: 40 mg.
Analyse d'acides aminés: Ala: 1,01 (1)
Val: 1 (1)
Sta: 2,03 (2)
Trp: 0,95 (1).

Spectre de RMN

| δ | Aspects | Intégration | Attribution | |
|---|---|---|---|---|
| 0,71—0,93 | m | 18 H | { $^6C(CH_3)_2$ | Sta |
| | | | $^3C(CH_3)_2$ | Val |
| 1,09—1,62 | m | 9 H | { $CH_3$ | Ala |
| | | | $^5CH_2$—$^6CH$ | Sta |
| 1,87—2,40 | m | 5 H | { $^3CH$ | Val |
| | | | $^2CH_2$ | Sta |
| 3,50 | s | 3 H | $CH_3$ | ester |
| 3,71—3,89 | m | 4 H | $^4CH$—$^3CH$ | Sta |
| 4,05—4,43 | m | 3 H | $^2CH$ | Trp, Val, Ala |
| 4,77—5,00 | m | 4 H | { 2 OH | Sta |
| | | | $CH_2$ | Z |
| 6,83—8,00 | m | 15 H | { aromatiques | |
| | | | 5×NH | |
| 10,72 | s | 1 H | NH | indole |

Exemple 17

Boc-(D)Phe-Val-Sta-Ala-Sta-OH (SR 41491)

76 mg de Boc-(D)Phe-Val-Sta-Ala-Sta-OMe (exemple 6) sont solubilisés dans 4 ml de DMF; ajouter 1 ml d'eau, puis, à TA, 0,15 ml de soude normale. Agiter 40 min à TA, puis ajouter 0,15 ml d'acide chlorhydrique normal. Evaporer les solvants sous haut vide au bain marie à 35°C. Reprendre le résidu dans l'eau. Essorer le solide, sécher. Laver à l'éther; sécher.

Rendement: 60 mg (80%)

Analyse d'acides aminés: Ala: 0,98 (1)
Val: 0,96 (1)
Phe: 0,97 (1)
Sta: 2,05 (2).

Exemple 18

Boc-Phe-His-Sta-Ala-Sta-OH (SR 41492)

60 mg de Boc-Phe-His-Sta-Ala-Sta-OMe (exemple 8) sont solubilisés dans 4 ml de DMF; ajouter 1 ml d'eau, puis à TA ajouter 0,12 ml de soude normale. Agiter 40 minutes à TA, puis ajouter 0,12 ml d'acide chlorhydrique normal. Evaporer les solvants sous haut vide au bain marie à 35°. Reprendre le résidu dans l'eau et le solide obtenu est essoré et séché.

Rendement: 30 mg

Analyse d'acides aminés: Ala: 1,01 (1)
Phe: 1,01 (1)
His: 0,96 (1)
Sta: 2,02 (2).

Exemple 19

Boc-(D)Phe-Phe-Sta-Ala-Sta-OMe (SR 41518)

150 me de H-Phe-Sta-Ala-Sta-OMe, TFA (exemple 1) et 37 mg de HOBt sont recouverts de 30 ml de dioxane contenant 57 mg de NEM. Ajouter 98 mg de Boc-(D)Phe-ONSu et agiter à TA. Contrôler le pH et ajuster à pH 6—7 par NEM, si nécessaire. Après 48 heures, évaporer le solvant, reprendre le résidu dans l'eau et le solide blanc obtenu est essoré, lavé à l'eau, puis à l'éther et séché.

Rendement: 150 mg (82%)

Analyse d'acides aminés: Ala: 1,0 (1)
Phe: 1,92 (2)
Sta: 2,07 (2).

Spectre de RMN

| δ | Aspects | Intégration | Attribution | |
|---|---|---|---|---|
| 0,71—0,86 | m | 12 H | $^6C(CH_3)_2$ | Sta |
| 1,07—1,59 | m | 18 H | $CH_3$ | Ala |
| | | | $(CH_3)_3C$ | Boc |
| | | | $^5CH_2—^6CH$ | Sta |
| 1,97—2,37 | m | 4 H | $^2CH_2$ | Sta |
| 2,37—2,62 | m | x | $^3CH_2$ | (D)Phe |
| 2,65—3,06 | m | 2 H | $^3CH_2$ | Phe |
| 3,50 | s | 3 H | $CH_3$ | ester |
| 3,67—3,90 | m | 4 H | $^4CH—^3CH$ | Sta |
| 4,02—4,15 | m | 2 H | $^2CH$ | (D)Phe |
| 4,15—4,28 | m | | $^2CH$ | Ala |
| 4,45—4,60 | m | 1 H | $^2CH$ | Phe |
| 4,76 | d | 1 H | 2×OH | Sta |
| 4,92 | d | 1 H | | |
| 6,58 | d | 1 H | NH | (D)Phe |
| 6,96—7,25 | m | 10 H | aromatiques | |
| 7,31 | d | 1 H | 2×NH | Sta |
| 7,45 | d | 1 H | | |
| 7,80 | d | 1 H | NH | Ala |
| 8,16 | d | 1 H | NH | Phe |
| x intégration impossible par suite du pic de DMSO à δ=2,45 | | | | |

Exemple 20

Adoc-Phe-Phe-Sta-Ala-Sta-OMe (SR 41540)

221 mg de H-Phe-Sta-Ala-Sta-OMe, TFA (exemple 1) et 54 mg de HOBt sont recouverts de 30 ml de dioxane contenant 84 mg de NEM. Ajouter 175 mg de Adoc-Phe-ONSu. Agiter à température ambiante. Contrôler le pH et ajuster à pH 6—7 par NEM, si nécessaire. Après 48 heures, évaporer le solvant sous vide, reprendre le résidu dans l'eau, essorer le solide, sécher. Le produit en solution dans méthanol-chloroforme 1/99 est déposé en tête d'une colonne de gel de silice 60 Merck$^R$ (70—230 Mesh) dans le même solvant (L: 40 cm — ϕ: 2 cm). Eluer avec.

— 200 ml de méthanol-chloroforme 1/99
— 300 ml de méthanol-chloroforme 2/98
— 300 ml de méthanol-chloroforme 5/95.

Fractionner et évaporer les fractions pures. Reprendre dans l'éther, essorer, sécher.

Rendement: 170 mg (58%)

Analyse d'acides aminés: Ala: 1,05 (1)
Sta: 1,97 (2)
Phe: 2,04 (2).

Spectre de RMN

| δ | Aspects | Intégration | Attribution | |
|---|---|---|---|---|
| 0,71—0,91 | m | 12 H | $^6C(CH_3)_2$ | Sta |
| 1,09—2,03 | m | 24 H | $CH_3$ | Ala |
| | | | $^5CH_2—^6CH$ | Sta |
| | | | $CH_2$ et CH | Adoc |
| 2,08—2,37 | m | — | $^2CH_2$ | Sta |
| 2,56—3,06 | m | — | $^3CH_2$ | Phe |
| 3,50 | s | — | $CH_3$ | ester |
| 3,68—3,87 | m | 4 H | $^4CH—^3CH$ | Sta |
| 3,94—4,58 | m | 3 H | $^2CH$ | Phe,Ala |
| 4,78 | d | — | OH | Sta |
| 4,93 | d | | | |
| 6,86 | d | 1 H | NH | |
| 7,06—7,26 | m | 10 H | aromatiques | |
| 7,34 | d | 1 H | NH | |
| 7,52 | d | 1 H | NH | |
| 7,78 | d | 1 H | NH | |
| 7,94 | d | 1 H | NH | |

Exemple 21

Boc-His(Boc)-Phe-Sta-Ala-Sta-OMe (SR 41541)

100 mg de H-Phe-Sta-Ala-Sta-OMe (exemple 1) et 75 mg de Boc-His(Boc)OH, DCHA sont solubilisés dans 20 ml de dioxane, contenant 10 ml de DMF. Ajouter 72 mg de BOP et 20,6 mg de DIPEA. Agiter à TA, ajuster le pH à 6—7 par DIPEA, si nécessaire. Après 48 heures, évaporer les solvants au bain-marie à 35° sous haut vide. Reprendre le résidu dans l'eau et extraire au chlorure de méthylène. La phase organique est séchée sur $Na_2SO_4$. Evaporer le solvant. Dissoudre dans le chloroforme et déposer en tête d'une colonne (L: 40 cm — ϕ: 2 cm) de gel de silice 60 Merck[R] (70—230 Mesh) dans le chloroforme. Eluer dans:

— 200 ml de chloroforme
— 200 ml de méthanol-chloroforme 1/99
— 200 ml de méthanol-chloroforme 2/98
— 100 ml de méthanol-chloroforme 3/97
— 300 ml de méthanol-chloroforme 4/96

Fractionner. Evaporer les fractions pures. Reprendre dans l'éther-hexane 50/50, essorer. Sécher.

Rendement 70 mg (60%)

Analyse d'acides aminés: Ala: 0,91 (1)
His: 1,20 (1)
Phe: 1,05 (1)
Sta: 1,80 (2).

Spectre de RMN

| δ | Aspects | Intégration | Attribution | |
|---|---|---|---|---|
| 0,69—0,83 | m | 12 H | $^6C\ (CH_3)_2$ | Sta |
| 1,09—1,54 | m | 27 H | $CH_3$ | Ala |
| | | | $(CH_3)_3C$ | diBoc |
| | | | $^5CH_2—^6CH$ | Sta |
| 1,94—2,37 | m | 4 H | $^2CH_2$ | Sta |
| 2,50—3,02 | m | 4 H | $^3CH_2$ | Phe, His |
| 3,50 | s | — | $^4CH—^3CH$ | Sta |
| 3,68—3,87 | m | 4 H | $^4CH—^3CH$ | Sta |
| 4,00—4,26 | m | 2 H | $^2CH$ | Phe, Ala, His |
| 4,43—4,59 | m | 1 H | | |
| 4,78 | d | — | 2×OH | Sta |
| 4,93 | d | | | |
| 6,85 | d | 1 H | NH | |
| 7,06—7,22 | m | 6 H | arom. | Phe |
| | | | 4′ CH | His |
| 7,36 | d | 1 H | NH | |
| 7,58 | d | 1 H | NH | |
| 7,81 | d | 1 H | NH | |
| 7,88 | d | 1 H | NH | |
| 8,03 | s | 1 H | 2′CH | His |

Exemple 22

Z-Tyr-Tyr-Sta-Ala-Sta-OMe (SR 41542)

1. Z-Tyr-Sta-Ala-Sta-OMe

Dans 25 ml de dioxane contenant 244 mg de NEM, on introduit successivement 400 mg de H-Sta-Ala-Sta-OMe, TFA, puis 310 mg de Z-Tyr-ONSu et 101 mg de HOBt. Agiter à TA, ajuster le pH à 6—7 par NEM, si nécessaire. Après 3 jours, évaporer le solvant au bain-marie à 40°, sous trompe à eau. Le résidu est repris dans l'eau et l'eau est décantée. Reprendre le résidu dans le chlorure de méthylène et le solide obtenu est essoré, lavé avec méthanol-chloroforme 2/98. Sécher.

Rendement: 340 mg (75%).

2. H-Tyr-Sta-Ala-Sta-OMe

180 mg de Z-Tyr-Sta-Ala-Sta-OMe sont dissous dans 50 ml de méthanol. Ajouter 40 mg de Pd/C 10% et hydrogéner sous une pression d'un mètre d'eau. Après une nuit, filtrer, laver le noir avec du méthanol, évaporer le solvant, reprendre le résidu dans l'éther et évaporer à sec. Reprendre dans l'éther, gratter et le solide obtenu est essoré. Sécher.

Rendement: 110 mg (96%).

3. Z-Tyr-Tyr-Sta-Ala-Sta-OMe

100 mg de H-Tyr-Sta-Ala-Sta-OMe, 82 mg de Z-Tyr-ONSu, 27 mg de HOBt sont solubilisés dans 30 ml de dioxane contenant 23 mg de NEM. Agiter à TA. Ajuster le pH à 6—7 par NEM, si nécessaire. Après 48

heures, évaporer le solvant, reprendre le résidu dans l'eau, triturer. Après 30 minutes, le solide obtenu est essoré, lavé à l'eau, séché. Laver à l'éther, puis au chlorure de méthylène. Dissoudre dans le méthanol, filtrer, évaporer le solvant, concentrer et précipiter par l'éther. Essorer, sécher.

Rendement: 100 mg (71%)

Analyse d'acides aminés: Ala: 1,03 (1)
Tyr: 1,92 (2)
Sta: 2,02 (2).

Spectre de RMN

| δ | Aspects | Intégration | Attribution | |
|---|---|---|---|---|
| 0,70—0,86 | m | 12 H | $^6C(CH_3)_2$ | Sta |
| 1,12—1,60 | m | 9 H | $CH_3$<br>$^5CH_2$—$^6CH$ | Ala<br>Sta |
| 1,95—2,37 | m | 4 H | $^2CH_2$ | Sta |
| 2,48—2,94 | m | 4 H | $^3CH_2$ | Tyr |
| 3,50 | s | — | $CH_3$ | ester |
| 3,70—3,89 | m | 4 H | $^4CH$—$^3CH$ | Sta |
| 4,00—4,48 | m | 3 H | $^2CH$ | Tyr, Ala |
| 4,75—4,96 | m | 4 H | 2×OH<br>$CH_2$ | Sta<br>Z |
| 6,51—6,63 | 2 d | 4 H | 3'5'CH | Tyr |
| 6,90—7,07 | 2 d | 4 H | 2'6'CH | Tyr |
| 7,14—7,50 | m | 8 H | aromatiques<br>3×NH | Z |
| 7,81 | d | 1 H | NH | |
| 8,00 | d | 1 H | NH | |
| 9,06<br>9,10 | s<br>s | — | 4'OH | Tyr |

Exemple 23

Boc-Pro-Pro-Sta-Ala-Sta-OMe (SR 41543)

1. Boc-Pro-Sta-Ala-Sta-OMe

Dans 70 ml de dioxane contenant 253 mg de NEM, on ajoute successivement 630 mg de H-Sta-Ala-Sta-OMe, TFA (exemple 1—4) puis 374 mg de Boc-Pro-ONSu et 162 mg de HOBt. Agiter à TA. Ajuster le pH à 6—7 par NEM si nécessaire. Après 48 heures, évaporer le solvant sous vide, reprendre le résidu dans l'eau. Extraire avec le chlorure de méthylène. Sécher sur $Na_2SO_4$, et évaporer le solvant. Dissoudre dans le chloroforme et déposer en tête d'une colonne (L: 40 cm — ϕ: 2 cm) de gel de silice 60 Merck$^R$ (70—230 Mesh) dans le chloroforme. Eluer avec:

— 200 ml de chloroforme
— 300 ml de méthanol-chloroforme 1/99
— 100 ml de méthanol-chloroforme 2/98
— 100 ml de méthanol-chloroforme 3/97
— 200 ml de méthanol-chloroforme 4/96
— 200 ml de méthanol-chloroforme 5/95.

Fractionner. Evaporer les fractions pures. Reprendre le résidu dans l'hexane. Essorer le solide. Sécher.
Rendement: 530 mg (85%).

2. H-Pro-Sta-Ala-Sta-OMe

500 mg de Boc-Pro-Sta-Ala-Sta-OMe sont recouverts de 8 ml de TFA. Après 30 minutes à TA, évaporer le solvant, reprendre le résidu dans l'éther, triturer, ajouter de l'hexane, essorer. Sécher.
Rendement: 510 mg (100%).

3. Boc-Pro-Pro-Sta-Ala-Sta-OMe (SR 41543)

125 mg de H-Pro-Sta-Ala-Sta-OMe, TFA sont recouverts de 35 ml de dioxane contenant 48 mg de NEM. Ajouter 69 mg de Boc-Pro-ONSu et 30 mg de HOBt. Agiter à TA. Ajuster le pH à 6—7 par NEM, si nécessaire. Après 48 heures, évaporer le solvant sous vide, reprendre le résidu dans l'eau et extraire au chlorure de méthylène. Sécher la phase organique sur $Na_2SO_4$ et évaporer à sec. Dissoudre le résidu dans le chloroforme et déposer en tête de colonne (L: 40 cm — ϕ: 2 cm) de gel de silice 60 Merck[R] (70—230 Mesh) dans le chloroforme. Eluer avec:

— 200 ml de chloroforme
— 200 ml de méthanol-chloroforme 1/99
— 200 ml de méthanol-chloroforme 2/98
— 200 ml de méthanol-chloroforme 3/97
— 300 ml de méthanol-chloroforme 5/95.

Fractionner et évaporer les fractions pures, reprendre dans l'éther évaporer l'éther à sec.
Rendement: 30 mg
Analyse d'acides aminés: Ala: 1 (1)
Pro: 1,54 (2)
Sta: 2,12 (2).

Spectre de RMN

| δ | Aspects | Intégration | Attribution | |
|---|---|---|---|---|
| 0,69—0,85 | m | 12 H | $^6C(CH_3)_2$ | Sta |
| 1,09—2,35 | m | 30 H | $CH_3$ | Ala |
| | | | $^5CH_2$—$^6CH$ | Sta |
| | | | $(CH_3)_3C$ | Boc |
| | | | β et γ $CH_2$ | Pro |
| | | | $^2CH_2$ | Sta |
| 3,18—3,65 | m | — | δ$CH_2$ | Pro |
| | | | $CH_3$ | ester |
| 3,65—3,89 | m | 4 H | $^4CH$—$^3CH$ | Sta |
| 4,08—4,21 | m | 1 H | $^2CH$ | Ala |
| 4,29—4,40 | m | 2 H | $^2CH$ | Pro |
| 4,72 | d | 1 H | OH | Sta |
| 4,86 | d | 1 H | OH | Sta |
| 7,30 | 2d | 2 H | 2×NH | Sta, Ala |
| 7,86 | d | 1 H | NH | |

Exemple 24

Adoc-Phe-Trp-Sta-Ala-Sta-OMe (SR 41581)

143 mg de H-Trp-Sta-Ala-Sta-OMe, TFA (exemple 5) sont recouverts de 30 ml de dioxane contenant 49

mg de NEM. Ajouter 97 mg de Acod Phe-ONSu et 30 mg de HOBt. Ajuster le pH à 6—7 par NEM, si nécessaire et agiter à TA. Après 48 heures, évaporer le solvant, reprendre le résidu dans l'eau, triturer et essorer le solide. Dissoudre le produit dans le chloroforme et déposer en tête d'une colonne (L: 60 cm — ϕ: 2 cm) de gel de silice 60 Merck[R] (70—230 mesh) dans le chloroforme. Eluer avec:

— 200 ml de chloroforme
— 200 ml de méthanol-chloroforme 1/99
— 200 ml de méthanol-chloroforme 3/97
— 200 ml de méthanol-chloroforme 5/95

Evaporer les fractions pures, reprendre dans l'éther, essorer, sécher.

Rendement: 120 mg (65%)

Analyse d'acides aminés: Ala: 0,95 (1)
Trp: 0,75 (1)
Phe: 0,99 (1)
Sta: 2,05 (2).

Spectre de RMN

| δ | Aspects | Intégration | Attribution | |
|---|---|---|---|---|
| 0,70—0,85 | m | 12 H | $^6C-(CH_3)_2$ | Sta |
| 1,14—2,42 | m | 28 H | $CH_3$ | Ala |
| | | | $^5CH_2-^6CH$ | Sta |
| | | | $CH_2$ et CH | Adoc |
| | | | $^2CH_2$ | Sta |
| 2,50—3,20 | m | | $^3CH_2$ | Phe, Trp |
| 3,50 | s | | $CH_3$ | ester |
| 3,73—3,92 | m | 4 H | $^4CH-^3CH$ | Sta |
| 3,95—4,61 | m | 3 H | $^2CH$ | Phe, Trp, Ala |
| 4,61 | d | — | OH | Sta |
| 4,96 | d | | | |
| 6,83—7,31 | m | 10 H | aromatiques | |
| 7,41 | d | 1 H | NH | |
| 7,46 | d | 1 H | NH | |
| 7,57 | d | 1 H | NH | |
| 7,81 | d | 1 H | NH | |
| 7,94 | d | 1 H | NH | |
| 10,64 | s | 1 H | NH | indole |

Exemple 25

Z-Phe-Pro-Sta-Ala-Sta-OMe (SR 41603)

150 mg de H-Pro-Sta-Ala-Sta-OMe, TFA (exemple 23-2) sont recouverts de 20 ml de dioxane contenant 59 mg de NEM, puis ajouter 110 mg de Z-Phe-ONSu et 37 mg de HOBt. Ajuster le pH à 6—7 par NEM, si nécessaire et agiter 48 heures à TA. Après 48 heures, évaporer le solvant, reprendre le résidu dans l'eau et extraire au chlorure de méthylène. Sécher sur $Na_2SO_4$, évaporer le solvant, puis dissoudre dans le

chloroforme et déposer en tête d'une colonne (L=60 cm—ϕ: 2 cm) de gel de silice 60 Merck[R] (70—230 Mesh) dans le chloroforme. Eluer avec:

— 200 ml de chloroforme
— 200 ml de méthanol-chloroforme 1/99
— 200 ml de méthanol—chloroforme 3/97
— 300 ml de méthanol-chloroforme 5/95

Evaporer les fractions contenant le produit.

Rendement: 100 mg (54%).

Spectre de RMN

| δ | Aspects | Intégration | Attribution | |
|---|---|---|---|---|
| 0,66—0,85 | m | 12 H | $^6C(CH_3)_2$ | Sta |
| 1,09—2,35 | m | 17 H | $CH_3$ | Ala |
| | | | $^5CH_2—^6CH$ | Sta |
| | | | β et γ $CH_2$ | Pro |
| | | | $^2CH_2$ | Sta |
| 2,50—3,02 | m | 2 H | $^3CH_2$ | Phe |
| 3,20—3,65 | m | — | δ$CH_2$ | Pro |
| | | | $CH_3$ | ester |
| 3,66—3,86 | m | 4 H | $^4CH—^3CH$ | Sta |
| 4,06—4,42 | m | 3 H | $^2CH$ | Phe, Pro, Ala |
| 4,72—4,93 | m | 4 H | 2 OH | Sta |
| | | | $CH_2$ | Z |
| 7,09—7,44 | m | 12 H | 10 aromatiques | |
| | | | 2×NH | |
| 7,63 | d | 1 H | NH | |
| 7,88 | d | 1 H | NH | |

Exemple 26

Boc-(D)Trp-Trp-Sta-Ala-Sta-OMe (SR 41604)

120 mg de H-Trp-Sta-Ala-Sta-OMe, TFA (exemple 5-) sont recouverts de 10 ml de dioxane contenant 41 mg de NEM. Ajouter 85 mg de Boc-(D) Trp-ONp et 27 mg de HOBt. Ajuster à pH 6—7 par NEM, si nécessaire et agiter 48 heures à TA. Evaporer le solvant, ajouter de l'eau, extraire au chlorure de méthylène, sécher celui-ci sur $Na_2SO_4$ et évaporer:dissoudre le résidu dans le chloroforme et déposer en tête d'une colonne (L=60 cm—ϕ: 2 cm) de gel de silice 60 Merck[R] (70—230 Mesh) dans le chloroforme. Eluer avec:

— 200 ml de chloroforme
— 200 ml de méthanol-chloroforme 1/99
— 200 ml de méthanol-chloroforme 2/98
— 200 ml de méthanol-chloroforme 3/97
— 200 ml de méthanol-chloroforme 4/96
— 300 ml de méthanol-chloroforme 5/95.

Fractionner. Evaporer les fractions contenant le produit, reprendre dans l'éther. Essorer, sécher.

Rendement: 60 mg (42%)

Analyse d'acides aminés: Ala: 0,99 (1)
Trp: 1,97 (2)
Sta: 2,04 (2).

| δ | Aspects | Intégration | Attribution | |
|---|---|---|---|---|
| 0,72—0,92 | m | 12 H | $^6C-(CH_3)_2$ | Sta |
| 1,12—1,60 | m | 18 H | { $CH_3$ | Ala |
| | | | $^5CH_2-^6CH$ | Sta |
| | | | $(CH_3)_3C$ } | Boc |
| 2,00—2,38 | m | 4 H | $^2CH_2$ | Sta. |
| 2,50—3,15 | m | — | $^3CH_2$ | Trp |
| 3,50 | s | — | $CH_3$ | ester |
| 3,73—3,91 | m | 4 H | $^4CH-^3CH$ | Sta |
| 4,08—4,59 | m | 3 H | { $^2CH$ | Ala |
| | | | $2\times{}^2CH$ } | Trp |
| 4,72 | d } | 2 H | 2×OH | Sta |
| 4,93 | d } | | | |
| 6,54 | d | 1 H | NH | |
| 6,80—7,47 | m | 11 H | { aromatiques | |
| | | | 1 NH } | |
| 7,57 | d | 1 H | NH | |
| 7,84 | d | 1 H | NH | |
| 8,08 | d | 1 H | NH | |
| 10,66 | s | 2 H | 2×NH | indole |

Exemple 27

Boc-Trp-His-Sta-Ala-Sta-OMe (SR 41619)

1. Boc-His-Sta-OH

2,63 g de Boc-His(Boc)-Sta-OMe (exemple 8-1) sont solubilisés dans 200 ml de DMF contenant 40 ml d'eau. La température étant à 25°, ajouter 3,15 g de baryte finement pulvérisée. Agiter et ajouter progressivement 150 ml d'eau froide pour éviter une élévation de température. Après 1 heure 30 à TA, faire barboter du gaz carbonique, puis filtrer et évaporer les solvants sous haut vide au bain-marie à 40°. Dissoudre le résidu dans le méthanol, filtrer, concentrer et verser sur de l'éther, nous avons obtenu un gel qui est essoré avec difficulté. Sécher.

Rendement: 1,23 g (60%).

2. Boc-His-Sta-Ala-Sta-OMe

360 mg de Boc-Ala-Sta-OMe (exemple 1-) sont recouverts de 3 ml de TFA. Après 30 min à TA, évaporer le solvant et dissoudre l'huile résiduelle dans le DMF; ajouter DIPEA jusqu'à obtention de pH 7—8 au papier pH, puis ajouter 412 mg de Boc-His-Sta-OH et 672 mg de BOP. Ajuster le pH à 6—7 par DIPEA, si nécessaire et agiter 48 heures à TA. Evaporer le solvant sous haut vide au bain-marie à 40°C. Reprendre le résidu dans du chloroforme et déposer en tête d'une colonne (L=40 cm — ϕ: 2 cm) de gel de silice 60 Merck[R] (70—230 Mesh) dans le chloroforme et éluer avec:

— 200 ml de chloroforme
— 200 ml de méthanol-chloroforme 1/99
— 200 ml de méthanol-chloroforme 2/98
— 200 ml de méthanol-chloroforme 3/97

— 200 ml de méthanol-chloroforme 4/96
— 400 ml de méthanol-chloroforme 5/95
— 400 ml de méthanol-chloroforme 6/94
— 200 ml de méthanol-chloroforme 10/90

et fractionner.

Evaporer les fractions contenant le produit, reprendre par l'éther, triturer et essorer le solide. Sécher.

Rendement: 350 mg (53%).

3. H-His-Sta-Ala-Sta-OMe, 2 TFA

220 mg de Boc-His-Sta-Ala-Sta-OMe sont recouverts de 3 ml de TFA. Après 30 min à TA, évaporer le solvant, reprendre le résidu dans l'éther, essorer le solide et sécher aussitôt sur $P_2O_5$.

Rendement: 235 mg (91%).

4. Boc-Trp-His-Sta-Ala-Sta-OMe (SR 41619)

235 mg de H-His-Sta-Ala-Sta-OMe, 2 TFA sont recouverts de 30 ml de dioxane contenant 68 mg de NEM. Ajouter 170 mg de Boc-Trp-ONp et 54 mg de HOBt. Ajuster le pH à 6—7 par NEM et agiter 48 heures à TA. Evaporer le solvant sous vide, reprendre le résidu dans l'eau, extraire au chlorure de méthylène et laver la phase organique avec de l'eau, puis $NaHCO_3$ 5% et à l'eau. Sécher sur $Na_2SO_4$. Evaporer le solvant. Dissoudre dans le chloroforme et disposer en tête d'une colonne (L=60 cm — ϕ: 2 cm) de gel de silice 60 Merck$^R$ (60—230 Mesh) dans le chloroforme.

Eluer avec:

— 100 ml de chloroforme
— 100 ml de méthanol-chloroforme 1/99
— 100 ml de méthanol-chloroforme 2/98
— 100 ml de méthanol-chloroforme 3/97
— 300 ml de méthanol-chloroforme 4/96
— 500 ml de méthanol-chloroforme 5/95
— 300 ml de méthanol-chloroforme 10/90

Fractionner. Evaporer les fractions contenant le produit, reprendre dans l'éther, essorer, sécher.

Rendement: 75 mg (32%)

Analyse d'acides aminés: Ala: 0,89 (1)
His: 0,80 (1)
Trp: 0,77 (1)
Sta: 2,31 (2).

Spectre de RMN

| δ | Aspects | Intégration | Attribution | |
|---|---|---|---|---|
| 0,50—1,57 | m | 30 H | $^6C(CH_3)_2$ | Sta |
| | | | $CH_3$ | Ala |
| | | | $(CH_3)_3C$ | Boc |
| | | | $^5CH_2—^6CH$ | Sta |
| 1,96—2,38 | m | 4 H | $^2CH_2$ | Sta |
| 2,70—3,16 | m | 4 H | $^3CH_2$ | Trp, His |
| 3,50 | s | 3 H | $CH_3$ | ester |
| 3,67—3,88 | m | 4 H | $^4CH—^3CH$ | Sta |
| 4,05—4,57 | m | 3 H | $^2CH$ | Trp, His, Ala |
| 4,82—5,00 | m | 2 H | OH | Sta |
| 6,72—7,59 | m | 10 H | aromatique | Trp, His |
| | | | 3×NH | |
| 7,95—8,20 | m | 2 H | 2×NH | |
| 10,74 | s | 1 H | NH | indole |

Exemple 28

Z-Tau-Phe-His-Sta-Ala-Sta-$OCH_3$ (SR 41748)

On dissout 2 TFA, H-His-Sta-Ala-Sta-$OCH_3$ (360 mg; exemple 27-3) dans 10 ml de DMF. La solution est amenée à pH 7 par addition de DIPEA. On y ajoute successivement 195 mg de Z-Tau-Phe-OH et 221 mg de BOP.

Le mélange réactionnel est amené à pH 7—8 par addition de DIPEA, agité 48 h à TA, puis évaporé à sec. Le produit obtenu est chromatographié sur une colonne de gel de silice (diamètre 2 cm, hauteur 30 cm) et élué avec

— 300 ml de chloroforme
— 200 ml de mélange chloroforme-méthanol 98-2
— 200 ml de mélange chloroforme-méthanol 97-3
— 200 ml de mélange chloroforme-méthanol 96-4
— 200 ml de mélange chloroforme-méthanol 95-5
— 400 ml de mélange chloroforme-méthanol 94-6
— 400 ml de mélange chloroforme-méthanol 92-8

Les bonnes fractions (déterminées par CCM) sont réunies et évaporées à sec, sous pression réduite à TA. On obtient une huile. Cette huile est purifiée de nouveau sur une colonne de gel de silice (diamètre 2 cm, hauteur 25 cm) et éluée avec

— 200 ml d'un mélange chloroforme-méthanol 99—1
— 200 ml d'un mélange chloroforme-méthanol 98,5—1,5
— 200 ml d'un mélange chloroforme-méthanol 98—2
— 200 ml d'un mélange chloroforme-méthanol 97,5—2,5
— 200 ml d'un mélange chloroforme-méthanol 97—3
— 100 ml d'un mélange chloroforme-méthanol 96—4
— 100 ml d'un mélange chloroforme-méthanol 94—6
— 100 ml d'un mélange chloroforme-méthanol 92—8
— 100 ml d'un mélange chloroforme-méthanol 90—10
— 100 ml d'un mélange chloroforme-méthanol 88—12
— 100 ml d'un mélange chloroforme-méthanol 86—14
— 100 ml d'un mélange chloroforme-méthanol 84—16.

Les bonnes fractions (déterminées par CCM) sont réunies et évaporées à sec. Le résidu est repris dans de l'acétate d'éthyle (20 ml). La phase organique est lavée avec une solution aqueuse saturée de bicarbonate de soude (10 ml), solution aqueuse de chlorure de sodium (10 ml), puis séchée sur sulfate de magnésium et évaporée à sec. Le résidu est repris dans l'éther, trituré, essoré. Poudre crème 60 mg.

CCM—chloroforme-méthanol-acide acétique 90—20—3 Rf=0,48.

Analyse d'acides aminées: Ala: 0,97
Sta: 1,99
Tau-Phe: 1,19
His: 0,86.

Spectre de RMN

| δ | Aspects | Intégration | Attribution | |
|---|---|---|---|---|
| 0,77 | m | 12 H | $^6C(CH_3)_2$ | Sta |
| 0,87—1,56 | m | 9 H | $^5CH_2—^6CH$ | Sta |
| | | | $CH_3$ | Ala |
| 1,91—2,4 | m | 4 H | $^2CH_2$ | Sta |
| 2,5—3,45 | m | 8 H (·) | $^3CH_2$ | Phe, His |
| | | | $^2CH_2—^3CH_2$ | Tau |
| 3,50 | s | 3 H | $CH_3$ | Ester |
| 3,61—3,88 | m | 4 H | $^3CH—^4CH$ | Sta |
| 4,05—4,55 | m | 3 H | $^2CH$ | Phe, Ala, His |
| 4,95 | massif | 4 H | $CH_2$ de | Z |
| | | | OH | Sta |
| 6,77 | s | 1 H | $^{4'}CH$ | His |
| 7,05—7,45 | m | 13 H | aromatique | |
| | | | 3 NH | |
| 7,46 | s | 1 H | $^{2'}CH$ | His |
| 7,66 | d | 1 H | NH | |
| 7,96 | d | 1 H | NH | |
| 8,14 | d | 1 H | NH | |

(·) Intégration perturbée par présence de DOH.

Exemple 29

iVa-Phe-Phe-Sta-Ala-Sta-OMe (SR 41764)

Dans 3 ml de dioxane, on ajoute successivement 130 mg de H-Phe-Sta-Ala-Sta-OMe (exemple 1—6), 79,5 mg de iVa-Phe-ONSu, 31 mg de HOBt et 75 mg de NEM. Agiter à TA. Ajuster le pH à 6—7 par NEM si nécessaire. Après 24 h, filtrer le solide, laver avec un peu de dioxane, puis à l'éther. Sécher.

Rendement: 110 mg, 73%

Analyse d'acides aminés: Ala: 1,03 (1)
Phe: 1,93 (2)
Sta: 2,04 (2).

Spectre de RMN

| δ | Aspects | Intégration | Attribution | |
|---|---|---|---|---|
| 0,51—0,93 | m | 18 H | $^6C(CH_3)_2$ | Sta |
| | | | $^3C(CH_3)_2$ | iVa |
| 1,09—1,88 | m | 12 H | $^5CH_2$—$^6CH$ | Sta |
| | | | $CH_3$ | Ala |
| | | | $^3CH$—$^2CH_2$ | iVa |
| 1,88—2,40 | m | 4 H | $^2CH_2$ | Sta |
| 2,50—3,09 | m | 4 H | $^2CH_2$ | Phe |
| 3,5 | s | 3 H | $CH_3$ | $COOCH_3$ |
| 3,66—4,59 | m | 7 H | $^4CH$—$^3CH$ | Sta |
| | | | $^2CH$ | Phe |
| | | | $^2CH$ | Ala |
| 4,8 | d | 1 H | OH | Sta |
| 4,95 | d | 1 H | OH | Sta |
| 7,01—7,27 | m | 10 H | aromatique | |
| 7,37 | d | 1 H | NH | |
| 7,46 | d | 1 H | NH | |
| 7,77 | d | 1 H | NH | |
| 7,88 | d | 1 H | NH | |

Exemple 30

iVa-Phe-Leu-Sta-Ala-Sta-OMe (SR 41765)

1. Boc-Leu-Sta-Alu-Sta-OMe

354 mg de H-Sta-Ala-Sta-OMe, TFA (exemple 1—3) sont recouverts de 30 ml de dioxane contenant 75 mg de NEM. Ajouter 260 mg de Boc-Leu-ONSu. Agiter à TA. Ajuster le pH à 6—7 si nécessaire, par NEM. Après 48 h, évaporer le solvant sous trompe à eau, au bain-marie à 40°C, reprendre le résidu dans l'eau glacée, extraire au chlorure de méthylène et sécher la phase organique sur $Na_2SO_4$. Evaporer. Dissoudre dans le chloroforme et déposer en tête d'une colonne (L: 40 cm, diamètre: 2 cm) de gel de silice 60 Merck[R] (70—230 mesh) dans le chloroforme. Eluer avec:

— 200 ml de chloroforme
— 200 ml de méthanol-chloroforme 1/99
— 200 ml de méthanol-chloroforme 2/98
— 200 ml de méthanol-chloroforme 3/98
— 200 ml de méthanol-chloroforme 4/98.

Fractionner, évaporer les fractions contenant le produit pur. Reprendre dans l'éther. Essorer. Sécher. Renedment: 160 mg 38%.

2. H-Leu-Sta-Ala-Sta-OMe, TFA

140 mg de Boc-Leu-Sta-Ala-Sta-OMe sont recouverts de 2 ml de TFA. Après 30 min à TA, évaporer le solvant, reprendre le résidu dans l'éther, essorer le solide obtenu. Sécher.

Rendement 140 mg, 100%.

3. iVa-Phe-Leu-Sta-Ala-Sta-OMe

110 mg de H-Leu-Sta-Ala-Sta-OMe, 79,5 mg de iVa-Phe-ONSu et 31 mg de HOBt sont recouverts de 3 ml de dioxane contenant 75 mg de NEM. Agiter à TA et ajuster le pH à 6—7 par NEM, si nécessaire.

Après 24 h, essorer le solide, laver au dioxane, puis à l'éther. Sécher.

Rendement: 80 mg, 55%

Analyse d'acides aminés: Ala: 1,02 (1)
Leu: 1,01 (1)
Phe: 0,9 (1)
Sta: 2,00 (2).

Spectre de RMN

| δ | Aspects | Intégration | Attribution | |
|---|---|---|---|---|
| 0,45—1,88 | m | 24 H | $^6C(CH_3)_2$ | Sta |
| | | | $^4C(CH_3)_2$ | Leu |
| | | | $^3C(CH_3)_2$ | iVa |
| 1,03—1,88 | m | 15 H | $^5CH_2$—$^6CH$ | Sta |
| | | | $CH_3$ | Ala |
| | | | $^3CH$—$^2CH_2$ | iVa |
| | | | $^4CH$—$^3CH_2$ | Leu |
| 1,95—2,5 | m | 4 H | $^2CH_2$ | Sta |
| 2,58—3,03 | m | 2 H | $^2CH_2$ | Phe |
| 3,5 | s | 3 H | $CH_3$ | $COOCH_3$ |
| 3,64—4,61 | m | 7 H | $^4CH$—$^3CH$ | Sta |
| | | | $^2CH$ | Phe |
| | | | $^2CH$ | Ala |
| | | | $^2CH$ | Leu |
| 7,04—7,24 | m | 5 H | aromatique | |
| 7,24—7,42 | m | 2 H | 2 NH | |
| 7,83 | d | 1 H | NH | |
| 7,90—8,09 | m | 2 H | 2 NH | |

Exemple 31

iVa-Phe-Phe-Sta-Ala-isoSta-OMe (SR 41766)

On opère comme dans l'exemple 29, en remplaçant, à la première étape, l'éther méthylique de la statine par son isomère l'isostatine.

De la même façon, on isole le produit attendu sous forme d'un solide.

Analyse d'acides aminés: Ala: 1,00 (1)
Phe: 2,00 (2)
Sta: 2,01 (2).

Spectre de RMN

| δ | Aspects | Intégration | Attribution | |
|---|---|---|---|---|
| 0,50—0,90 | m | 18 H | $^6C(CH_3)_2$ | iso Sta |
| | | | $^6C(CH_3)_2$ | Sta |
| | | | $^3C(CH_3)_2$ | iVa |
| 1,04—1,85 | m | 12 H | $^5CH_2—^6CH$ | Sta |
| | | | $^5CH_2—^6CH$ | iso Sta |
| | | | $CH_3$ | Ala |
| | | | $^3CH—^2CH_2$ | iVa |
| 1,91—2,5 | m | 4 H | $^2CH_2$ | Sta |
| | | | $^2CH_2$ | iso Sta |
| 2,5—3,08 | m | 4 H | $^2CH_2$ | Phe |
| 3,51 | s | 3 H | $CH_3$ | $COOCH_3$ |
| 3,54—4,62 | m | 7 H | $^4CH—^3CH$ | Sta |
| | | | $^4CH_3—CH$ | iso Sta |
| | | | $^2CH$ | Phe |
| | | | $^2CH$ | Ala |
| 4,80 | d | 1 H | OH | Sta |
| 4,91 | d | 1 H | OH | iso Sta |
| 7,01—7,25 | m | 10 H | aromatique | |
| 7,41 | d | 1 H | NH | |
| 7,53 | d | 1 H | NH | |
| 7,77 | d | 1 H | NH | |
| 7,91 | d | 1 H | NH | |
| 8,11 | d | 1 H | NH | |

Exemple 32

$C_6H_5CH_2CH_2CO$-Phe-Phe-Sta-Ala-Sta-OMe (SR 41768)

Dans 3 mg de dioxane contenant 75 mg de NEM, on introduit successivement 130 mg de H-Phe-Sta-Ala-Sta-OMe, TFA (exemple 1—6), 91 mg de $C_6H_5$-$CH_2$-$CH_2$-CO-Phe-ONSu et 31 mg de HOBt. Agiter à TA. Ajuster le pH à 6—7 par NEM si nécessaire. Après 24 h, essorer le solide, laver au dioxane, puis à l'éther. Sécher.

Rendement: 115 mg (71).

Analyse d'acides aminés: Ala: 1,01 (1)
Phe: 1,99 (2)
Sta: 2,00 (2).

Spectre de RMN

| δ | Aspects | Intégration | Attribution | |
|---|---|---|---|---|
| 0,69—0,87 | m | 12 H | $^6C(CH_{32}$ | Sta |
| 1,08—1,59 | m | 9 H | $^5CH_2—^6CH$ | Sta |
| | | | $CH_3$ | Ala |
| 1,88—2,40 | m | 8 H | $^2CH_2$ | Sta |
| | | | $^3CH_2—^2CH_2$ | $C_6H_5$ \| $(CH_2)_2$ |
| 2,48—3,11 | m | 4 H | $CH_2$ | Phe |
| 3,5 | s | 3 H | $CH_3$ | $COOCH_3$ |
| 3,67—4,56 | m | 7 H | $^4CH—^3CH$ | Sta |
| | | | $^2CH$ | Phe |
| | | | $^2CH$ | Ala |
| 4,79 | d | 1 H | OH | Sta |
| 4,93 | d | 1 H | OH | Sta |
| 6,98—7,25 | m | 15 H | aromatique | |
| 7,35 | d | 1 H | NH | |
| 7,45 | d | 1 H | NH | |
| 7,77 | d | 1 H | NH | |
| 7,98 | d | 1 H | NH | |
| 8,14 | d | 1 H | NH | |

Exemple 33
iVa-Phe-Nle-Sta-Ala-Sta-OMe (SR 41919)

1. Boc-Nle-Sta-Ala-Sta-OMe

531 mg de H-Sta-Ala-Sta-OMe, TFA (exemple 1—4), 393 mg de Boc-Nle-ONSu et 162 mg de HOBt sont recouverts de 5 ml de dioxane contenant 391 mg de NEM. Ajuster le pH à 6—7 par NEM si nécessaire et agiter 48 h à TA. Evaporer le solvant, dissoudre le résidu dans ACOEt et laver successivement par $KHSO_4—K_2SO_4$ 5%, eau/ClNa, $NaHCO_3$ 5%, eau/ClNa. Sécher sur $MgSO_4$, évaporer le solvant. Dissoudre le résidu dans l'éther et précipiter le produit par le pentane. Essorer. Sécher.

Rendement: 570 mg, 90%.

2. H-Nle-Sta-Ala-Sta-OMe, TFA

570 mg de Boc-Nle-Sta-Ala-Sta-OMe sont recouverts de 5 ml de TFA. Après 15 min à TA, évaporer le solvant et reprendre le résidu dans un mélange éther-hexane 1/1. Essorer le solide, sécher sous vide.

Rendement: 570 mg, 100%.

3. iVa-Phe-Nle-Sta-Ala-Sta-OMe (SR 41919)

Dans 3 ml dioxane contenant 89 mg de NEM, on ajoute 150 mg de H-Nle-Sta-Ala-Sta-OMe, TFA, 36 mg de HOBt et 94 mg de iVa-Phe-ONSu. Agiter à TA. Ajuster le pH à 6—7 par NEM si nécessaire. Après 24 h, essorer le solide, laver au dioxane puis à l'éther, sécher.

Rendement: 130 mg, 73%.

Analyse d'acides aminés: Ala: 1,02 (1)
Nle: 1,01 (1)
Sta: 1,95 (2)
Phe: 1,01 (1).

*Spectre de RMN*

| δ | Aspects | Intégration | Attribution | |
|---|---|---|---|---|
| 0,59—0,91 | m | 21 H | $^6C(CH_3)_2$ | Sta |
| | | | $CH_3$ | Nle |
| | | | $^3C(CH_3)_2$ | iVa |
| 1,09—1,95 | m | 18 H | $^5CH_2$—$^6CH$ | Sta |
| | | | $CH_3$ | Ala |
| | | | $^3CH$—$^2CH_2$ | iVa |
| | | | $CH_2$—$CH_2$—$CH_2$ | Nle |
| 1,95—2,38 | m | 4 H | $^2CH_2$ | Sta |
| 2,56—3,08 | m | 2 H | $^3CH_2$ | Phe |
| 3,53 | s | 3 H | $CH_3$ | $COOCH_3$ |
| 3,62—4,59 | m | 7 H | $^4CH^3$—CH | Sta |
| | | | $^2CH$ | Phe |
| | | | $^2CH$ | Ala |
| | | | $^2CH$ | Nle |
| 4,83 | d | 1 H | OH | Sta |
| 4,95 | d | 1 H | OH | |
| 7,01—7,29 | m | 5 H | aromatique | |
| 7,37 | 2 d | 2 H | 2 NH | |
| 7,85 | d | 1 H | NH | |
| 8 | 2 d | 2 N | 2 NH | |

Exemple 34

$C_6H_5SO_2$-Phe-Phe-Sta-Ala-Sta-$OCH_3$ (SR 41938)

On dissout 300 mg de TFA, H-Phe-Sta-Ala-Sta-$OCH_3$ (exemple 1—6) dans 25 ml de DMF. La solution est amenée à pH 7 par addition de DIPEA. A cette solution, on ajoute successivement:

146 mg de $C_6H_5$-$SO_2$-Phe-OH
99 mg de DCCI
74 mg de HOBt (produit contenant 13% d'eau)

puis le pH est amené à 7 par addition de DIPEA. On agite une nuit à TA, puis le mélange réactionnel est évaporé à sec sous pression réduite. Le résidu est repris dans 20 ml de chlorure de méthylène. On essore le DCU formé. La phase chlorure de méthylène est lavée successivement:

— avec une solution aqueuse saturée de bicarbonate de soude (2×10 ml)
— avec une solution aqueuse de $SO_4HK$-$SO_4K_2$ (pH 2) (2×10 ml)
— avec de l'eau (2×10 ml)

puis séchée sur sulfate de magnésium et évaporée à sec sous pression réduite. Le résidu est repris dans un peu d'éther et essoré; poudre blanche 300 mg.

Le produit brut est alors purifié par chromatographie sur une colonne de gel de silice (diamètre: 2 cm, hauteur 25 cm) et élué avec

— chloroforme 300 ml
— mélange chloroformé-méthanol 80-20:100 ml
— mélange chloroforme-méthanol 60-40:100 ml
— mélange chloroforme-méthanol 40-60:100 ml.

Les diverses fractions sont déterminées par CCM.
On recueille:

— une fraction A (10 mg) qui est éliminée
— une fraction B.

après évaporation des solvants et reprise dans l'éther on obtient 130 mg d'une poudre blanche (RT 34,5%).
CCM—chloroforme-méthanol-acide acétique 90-20-3—Rf=0,71.

Analyse pondérale

| | | | |
|---|---|---|---|
| Calculé: | C 62,02; | H 7,21; | N 8,22 |
| Trouvé: | C 62,07; | H 7,03; | N 8,17 |

Spectre de RMN

| δ | Aspects | Intégration | Attribution | |
|---|---|---|---|---|
| 0,76 | m | 12 H | $^2C(CH_3)_2$ | Sta |
| 0,92—1,59 | m | 9 H | { $^5CH_2$—$^6CH$ | Sta |
| | | | { $CH_3$ | Ala |
| 1,8—2,38 | m | 4 H | $^2CH_2$ | Sta |
| 2,5—3,05 | m | 4 H | $^3CH_2$ | Phe |
| 3,50 | s | 3 H | $CH_3$ | ester |
| 3,62—3,90 | m | 4 H | $^3CH$—$^4CH$ | Sta |
| 4,00 | m | 1 H } | $^2CH$ | Phe, Phe, Ala |
| 4,21 | m | 2 H } | | |
| 4,75 | d | 1 H | OH | Sta |
| 4,93 | d | 1 H | OH | Sta |
| 6,69—7,49 | m | 17 H | { aromatiques | |
| | | | { 2NH | |
| 7,74 | d | 1 H | NH | |
| 7,93 | se (singulet élargi) | 1 H | NH | |
| 8,19 | d | 1 H | NH | |

Exemple 35

$C_6H_5CH_2SO_2$-Phe-Phe-Sta-Ala-Sta-$OCH_3$ (SR 41939)

On opère comme dans l'exemple 34 en remplaçant $C_6H_5SO_2$-Phe-OH par une quantité équivalente de $C_6H_5CH_2SO_2$-Phe-OH.

De la même façon, on isole le produit attendu.

Spectre de RMN

| δ | Aspects | Intégration | Attribution | |
|---|---|---|---|---|
| 0,74 | m | 12 H | $^6C(CH_3)_2$ | Sta |
| 0,93—1,53 | m | 9 H | $^5CH_2—^6CH$ | Sta |
| | | | $CH_3$ | Ala |
| 1,85—2,37 | m | 4 H | $^2CH_2$ | Sta |
| 2,54—3,13 | m | 4 H | $^3CH_2$ | Phe |
| 3,43—3,67 | m | 5 H | $CH_3$ | ester |
| | | | $CH_2$ de $C_6H_5—CH_2—SO_2—$ | |
| 3,67—3,88 | m | 4 H | $^3CH—^4CH$ | Sta |
| 4,04 | m | 1 H | | |
| 4,20 | m | 1 H | $^2CH$ | Phe, Phe, Ala |
| 4,61 | m | 1 H | | |
| 4,80 | d | 1 H | OH | Sta |
| 4,94 | d | 1 H | OH | Sta |
| 6,93—7,66 | m | 18 H | aromatiques | |
| | | | 3 NH | |
| 7,79 | d | 1 H | NH | |
| 8,37 | d | 1 H | NH | |

Exemple 36

Ac-Phe-Nva-Sta-Ala-Sta-OMe (SR 41994)

1. Boc-Nva-Sta-Ala-Sta-OMe

Dans 50 ml d'ACOEt contenant 391 mg de NEM, on ajoute successivement 531 mg de H-Sta-Ala-Sta-OMe, TFA (exemple 1—4), 162 mg de HOBt et 378 mg de Boc-Nva-ONSu. Agiter à TA, ajuster le pH à 6—7 par NEM si nécessaire. Après 24 h, laver successivement la solution organique par $KHSO_4—K_2SO_4$ 5%, ClNa/eau, NaH $CO_3$ 5%, ClNa/eau. Sécher sur $MgSO_4$, évaporer le solvant, reprendre l'huile résiduelle dans l'éther et laisser au repos: un précipité se forme. Essorer, laver avec un peu d'éther, sécher.

Rendement: 320 mg, 51%.

2. H-Nva-Sta-Ala-Sta-OMe, TFA

300 mg de Boc-Nva-Sta-Ala-Sta-OMe sont recouverts de 2 ml de TFA. Après 20 min à TA, évaporer le solvant, reprendre le résidu dans l'éther, essorer, sécher.

Rendement: 300 mg, 100%.

3. Ac-Phe-Nva-Sta-Ala-Sta-OMe

Dans 5 ml d'ACOEt contenant 58 mg de NEM, on ajoute successivement 100 mg de H-Nva-Sta-Ala-Sta-OMe, TFA, 23 mg HOBt et 53 mg de Ac-Phe-ONsu. Agiter à TA, ajuster le pH à 6—7 par NEM si nécessaire. Après 24 h, essorer le solide, laver avec ACOEt, puis à l'éther. Sécher.

Rendement: 70 mg, 63%.

Analyse d'acide aminé: Ala: 1,00 (1)
Nva: 1,00 (1)
Sta: 2,00 (2)
Phe: 1,00 (1).

Spectre de RMN

| δ | Aspects | Intégration | Attribution | |
|---|---|---|---|---|
| 0,64—0,93 | m | 15 H | $^{6}C(CH_3)_2$ | Sta |
| | | | $CH_3$ | Nva |
| 1,08—1,83 | m | 16 H | $^{5}CH_2—^{6}CH$ | Sta |
| | | | $CH_3$ | Ala |
| | | | $CH_2—CH_2$ | Nva |
| | | | $CH_3$ | Ac |
| 1,96—2,41 | m | 4 H | $^{2}CH_2$ | Sta |
| 2,56—3,04 | m | 2 H | $^{2}CH_2$ | Phe |
| 3,5 | s | 3 H | $CH_3$ | $COOCH_3$ |
| 3,67—4,56 | m | 7 H | $^{4}CH—^{3}CH$ | Sta |
| | | | $^{2}CH$ | Phe |
| | | | $^{2}CH$ | Ala |
| | | | $^{2}CH$ | Nva |
| 4,83 | d | 1 H | OH | Sta |
| 4,96 | d | 1 H | OH | Sta |
| 7,08—7,32 | m | 5 H | aromatique | |
| 7,32—7,50 | m | 2 H | 2 NH | |
| 7,95 | d | 1 H | NH | |
| 8,08—8,25 | m | 2 H | 2 NH | |

Exemple 37

iVa-Phe-Nva-Sta-Ala-Sta-OMe (SR 41995)

On opère comme dans l'exemple 36-3 en remplaçant Ac-Phe-ONSu par une quantité équivalente de iVa-Phe-ONSu.

De la même façon, on obtient le produit attendu.

Analyse d'acides aminés: Ala: 1,02 (1)
Nva: 1,00 (1)
Sta: 1,96 (2)
Phe: 1,02 (1).

Spectre de RMN

| δ | Aspects | Intégration | Attribution | |
|---|---|---|---|---|
| 0,54—0,91 | m | 21 H | $^6C(CH_3)_2$ | Sta |
| | | | $^3C(CH_3)_2$ | iVa |
| | | | $CH_3$ | Nva |
| 1,08—1,88 | m | 16 H | $^5CH_2—^6CH$ | Sta |
| | | | $CH_3$ | Ala |
| | | | $^3CH—^2CH_2$ | iVa |
| | | | $CH_2—CH_2$ | Nva |
| 1,96—2,37 | m | 4 H | $^2CH_2$ | Sta |
| 2,56—3,03 | m | 2 H | $^2CH_2$ | Phe |
| 3,5 | s | 3 H | $CH_3$ | $COOCH_3$ |
| 3,67—4,59 | m | 7 H | $^4CH—^3CH$ | Sta |
| | | | $^2CH$ | Phe |
| | | | $^2CH$ | Ala |
| | | | $^2CH$ | Nva |
| 4,82 | d | 1 H | OH | Sta |
| 4,95 | d | 1 H | OH | Sta |
| 7,03 | m | 5 H | aromatique | |
| 7,35 | 2 d | 2 H | 2 NH | |
| 7,85 | d | 1 H | NH | |
| 7,93—8,08 | m | 2 H | 2 NH | |

Exemple 38

Boc-Phe-Nva-Sta-Ala-Sta-OMe (SR 41996)

On obtient ce produit comme dans l'exemple 36-3 en remplaçant Ac-Phe-ONSu par Boc-Phe-ONSu en quantité équivalente.

Analyse d'acides aminés: Ala: 0,99 (1)
Nva: 1,00 (1)
Sta: 2,01 (2)
Phe: 1,01 (1).

Spectre de RMN

| δ | Aspects | Intégration | Attribution | |
|---|---|---|---|---|
| 0,69—0,90 | m | 15 H | $^6C(CH_3)_2$ | Sta |
| | | | $CH_3$ | Nva |
| 1,06—1,69 | m | 22 H | $^5CH_2—^6CH$ | Sta |
| | | | $CH_3$ | Ala |
| | | | $C(CH_3)_3$ | Boc |
| | | | $CH_2—CH_2$ | Nva |
| 1,95—2,40 | m | 4 H | $^2CH_2$ | Sta |
| 2,53—3,00 | m | 2 H | $^2CH_2$ | Phe |
| 3,51 | s | 3 H | $CH_3$ | $COOCH_3$ |
| 3,64—4,32 | m | 7 H | $^4CH—^3CH$ | Sta |
| | | | $^2CH$ | Nva |
| | | | $^2CH$ | Ala |
| | | | $^2CH$ | Phe |
| 4,81 | d | 1 H | OH | Sta |
| 4,95 | d | 1 H | OH | Sta |
| 6,95 | d | 1 H | NH | |
| 7,06—7,30 | m | 5 H | aromatique | |
| 7,35 | d | 1 H | NH | |
| 7,45 | d | 1 H | NH | |
| 7,90 | 2 d | 2 H | 2 NH | |

Exemple 39

Boc-Ile-His-Sta-Ala-Sta-OMe (SR 42019)

1. Boc-Ile-His-Sta-OMe

710 mg de Boc-His-(Boc)-Sta-OMe (exemple 8-1) sont recouverts de 8 ml de TFA. Après 15 min à TA, évaporer le solvant, reprendre le résidu dans l'éther et évaporer. L'huile résiduelle est solubilisée dans 10 ml de DMF et on ajoute DIPEA jusqu'à l'obtention de pH 6—7, puis on ajoute une solution de 475 mg de Boc-Ile-OH dans 10 ml de DMF contenant 258 mg de DIPEA, puis une solution de 986 mg de BOP dans 10 ml de DMF contenant 287 mg de DIPEA. Agiter à TA, ajuster le pH à 6—7 par DIPEA, si nécessaire. Après 24 h, évaporer le DMF sous haut vide, au bain-marie à 40°C, reprendre le résidu dans AcOEt et laver celui-ci par $HNa\ CO_3$ 5%, ClNa/eau. Sécher sur $MgSO_4$ · Evaporer. Dissoudre le produit dans MeOH-chloroforme 5—95 et déposer en tête d'une colonne de gel de silice 60 Merck (70—230 mesh) (L: 40 cm, diamètre: 3 cm). Eluer en faisant un gradient de MeOH jusqu'à 20—50. Evaporer les fractions contenant le produit pur. Reprendre le résidu dans l'eau, essorer. Sécher.

Rendement: 430 mg, 43%.

CCM—chloroforme-MeOH-acide acétique 80/15/5.

2. Boc-Ile-His-Sta-OH

410 mg de Boc-Ile-His-Sta-OMe sont solubilisés dans 25 ml de DMF. Ajouter 2 ml d'eau distillée, puis à TA, 1,5 ml de soude normale. Agiter 30 min à TA, puis ajouter 1,5 ml d'acide chlorhydrique normal. Evaporer les solvants, reprendre le résidu dans un minimum d'eau, essorer, sécher. Laver à l'éther, sécher.

Rendement: 320 mg, 81%.

3. Boc-Ile-His-Sta-Ala-Sta-OMe

180 mg de Boc-Ala-Sta-OMe sont recouverts de 3 ml de TFA. Après 30 min à TA, évaporer le solvant à fond. Dissoudre le résidu dans 30 ml de dioxane et amener à pH 7—8 par DIPEA. Ajouter 262 mg de Boc-Ile-His-Sta-OH, puis 268 mg de BOP. Ajuster le pH à 6—7 par DIPEA. Agiter à TA et contrôler plusieurs fois le pH. Après 24 h, filtrer, évaporer le solvant. Dissoudre dans le chloroforme et déposer en tête d'une colonne (L: 40 cm, diamètre: 2 cm) de gel de silice 60 Merck (70—230 mesh) dans le chloroforme. Eluer avec:

200 ml de chloroforme
200 ml MeOH-chloroforme 1—99
200 ml MeOH-chloroforme 2—98
400 ml MeOH-chloroforme 4—96
800 ml MeOH-chloroforme 6—94
200 ml MeOH-chloroforme 10—90.

Fractionner. Evaporer les fractions contenant le produit pur. Reprendre le résidu dans un peu de chlorure de méthylène, essorer, rincer à l'éther. Sécher.

Rendement: 80 mg, 24%.

Analyse d'acides aminés: Ala: 1,02 (1)
Ile: 0,97 (1)
Sta: 1,98 (2)
His: 1,00 (1).

Spectre de RMN

| δ | Aspects | Intégration | Attribution | |
|---|---|---|---|---|
| 0,64—0,88 | m | 18 H | $^{6}C(CH_3)_2$ | Sta |
| | | | $^{2}CH_3$ | Ile |
| 0,91—1,70 | m | 21 H | $CH_3$ | Ala |
| | | | $^{5}CH_2$—$^{6}CH$ | Sta |
| | | | $^{4}CH_2$—$^{3}CH$ | Ile |
| | | | Boc | |
| 1,88—2,41 | m | 4 H | $^{2}CH_2$ | Sta |
| 2,66—2,95 | m | 2 H | $^{3}CH_2$ | His |
| 3,50 | s | 3 H | $CH_3$ | Ester |
| 3,61—3,91 | m | 5 H | $^{4}CH$—$^{3}CH$ | Sta |
| | | | $^{2}CH$ | Ile |
| 4,09—4,25 | m | 1 H | $^{2}CH$ | Ala |
| 4,38—4,56 | m | 1 H | $^{2}CH$ | His |
| 4,74—5,12 | m | | OH | Sta |
| | | | OH | |
| 6,74—6,87 | (d+s) | 2 H | $^{4'}CH$ | His |
| | | | NH | |
| 7,24—7,5 | 2 d | 2 H | 2 NH | |
| 7,67 | s | 1 H | $^{2'}CH$ | His |
| 7,87—8,06 | 2 d | 2 H | 2 NH | |

Exemple 40

$CH_3(CH_2)_6$-CO-Phe-Phe-Sta-Ala-StaOH (SR 42062)

1. $CH_3(CH_2)_6$-CO-Phe-Phe-Sta-Ala-Sta-OMe

On prépare ce produit comme indiqué dans l'exemple 29 en remplaçant iVa-Phe-ONSu par $CH_3$-$(CH_2)_6$-CO-Phe-ONSu.

2. $CH_3(CH_2)_6$-CO-Phe-Phe-Sta-Ala-Sta-OH

150 mg de $CH_3(CH_2)_6$-CO Phe-Phe-Sta-Ala-Sta-OMe sont solubilisés dans 10 ml de DMSO, auxquels on ajoute 2 ml d'eau distillée et 2 ml de méthanol, puis à TA 1 ml de soude normale. Agiter 4 h à TA, puis ajouter 1 ml d'acide chlorhydrique normal: le pH est alors à 5. Evaporer les solvants sous haut vide au bain-marie à 40°C et reprendre le DMSO restant dans l'eau. Essorer le solide, laver à l'eau. Sécher. Laver à l'éther.

Rendement: 130 mg, 88%.

Analyse d'acides aminés: Ala: 0,99 (1)
Sta: 1,94 (2)
Phe: 2,03 (2).

Spectre de RMN

| δ | Aspects | Intégration | Attribution | |
|---|---|---|---|---|
| 0,69—0,87 | m | 15 H | $^6C(CH_3)_2$ | Sta |
| | | | $CH_3$ | Octyl |
| 0,87—1,61 | m | 21 H | $^5CH_2$—$^6CH$ | Sta |
| | | | $CH_3$ | Ala |
| | | | —$(CH_2)_6$ | octyl |
| 1,74—2,29 | m | 4 H | $^2CH_2$ | Sta |
| 2,51—3,06 | m | 4 H | $^3CH_2$ | Phe |
| 3,64—4,56 | m | 7 H | $^4CH$—$^3CH$ | Sta |
| | | | $^2CH$ | Phe |
| | | | $^2CH$ | Ala |
| 7,03—7,24 | m | 10 H | aromatique | |
| 7,33 | d | 1 H | NH | |
| 7,43 | d | 1 H | NH | |
| 7,75 | d | 1 H | NH | |
| 7,87 | d | 1 H | NH | |
| 8,06 | d | 1 H | NH | |

Exemple 41

iVa-Phe-Nle-Sta-Ala-Sta-OH (SR 42128)

100 mg de iVa-Phe-Nle-Sta-Ala-Sta-OMe (exemple 33-3) sont solubilisés dans 10 ml de DMF à TA. Ajouter 1 ml d'eau distillée, puis à TA 0,26 ml de soude normale (2 équivalents). Agiter 30 min à TA puis ajouter 0,26 ml d'acide chlorhydrique normal, le pH est alors à 5. Evaporer les solvants, reprendre le résidu dans l'eau; essorer le solide, laver à l'eau, puis à l'éther. Sécher.

Rendement: 75 mg.

Analyse d'acides aminés: Ala: 0,99 (1)
Nle: 1,00 (1)
Sta: 2,04 (2)
Phe: 0,98 (1).

Spectre de RMN

| δ | Aspects | Intégration | Attribution | |
|---|---|---|---|---|
| 0,58—0,91 | m | 21 H | $^{6}C(CH_3)_2$ | Sta |
| | | | $CH_3$ | Nle |
| | | | $^{3}C(CH_3)_2$ | iVa |
| 1,04—1,93 | m | 18 H | $^{5}CH_2—^{6}CH$ | Sta |
| | | | $CH_3$ | Ala |
| | | | $^{2}CH_2—^{3}CH$ | iVa |
| | | | $—CH_2—CH_2—CH_2$ | Nle |
| 1,93—2,29 | m | 4 H | $^{2}CH_2$ | Sta |
| 2,56—3,04 | m | 2 H | $^{3}CH_2$ | Phe |
| 3,64—4,61 | m | 7 H | $^{4}CH—^{3}CH$ | Sta |
| | | | $^{2}CH$ | Phe |
| | | | $^{2}CH$ | Ala |
| | | | $^{2}CH$ | Nle |
| 7,06—7,29 | m | 5 H | aromatique | |
| 7,29—7,45 | m | 2 H | 2 NH | |
| 7,83 | d | 1 H | NH | |
| 8 | 2 d | 2 H | NH | |

Exemple 42

Boc-Phe-Phe-Sta-Ala-Sta-OH, sel d'arginine

1. Boc-Phe-Phe-Sta-Ala-Sta-OH

270 mg de Boc-Phe-Phe-Sta-Ala-Sta-OMe (exemple 4) sont solubilisés dans 10 ml de DMF à TA. Ajouter 1 ml d'eau distillée, puis 0,66 ml de soude normale. Agiter 30 min à TA, puis ajouter 0,66 ml d'acide chlorhydrique normal; le pH est alors à 6 au papier pH. Evaporer les solvants sous haut vide. Reprendre le résidu dans l'eau, triturer, essorer le solide, laver à l'eau, puis à l'éther. Sécher.

Rendement: 230 mg, 87%.

Analyse d'acides aminés: Ala: 0,96 (1)
Sta: 2,00 (2)
Phe: 2,04 (2).

Spectre de RMN

| δ | Aspects | Intégration | Attribution | |
|---|---|---|---|---|
| 0,65—0,95 | m | 12 H | $^6C(CH_3)_2$ | Sta |
| 0,95—1,68 | m | 18 H | $^5CH_2$—$^6CH$ | Sta |
| | | | $CH_3$ | Ala |
| | | | Boc | Phe |
| 1,93—2,45 | m | 4 H | $^2CH_2$ | Sta |
| 2,54—3,14 | m | 4 H | $^3CH_2$ | Phe |
| 3,66—3,90 | m | 4 H | $^4CH$—$^3CH$ | Sta |
| 3,90—4,30 | m | 2 H | $^2CH$ | Phe |
| 4,46—4,66 | m | 1 H | $^2CH$ | Ala |
| 6,85 | d | 1 H | NH | |
| 7,04—7,27 | m | 10 H | aromatique | |
| 7,36 | d | 1 H | NH | |
| 7,57 | d | 1 H | NH | |
| 7,80 | d | 1 H | NH | |
| 7,96 | d | 1 H | NH | |

2. Sel. d'arginine

34,8 mg de H-Arg-OH anhydre sont solubilisés dans 3 ml d'eau distillée et, à cette solution, on ajoute une solution de 159,4 mg de Boc-Phe-Phe-Sta-Ala-Sta-OH dans 10 ml de méthanol. Agiter à TA durant 15 min et évaporer à sec. Reprendre le résidu dans l'éther, essorer, sécher sous vide.

Rendement: 170 mg (89%).

Exemple 43

iVa-Phe-Lys(Z)-Sta-Ala-Sta-OMe (SR 42130)

1. Boc-Lys(Z)-Sta-OEt

Dans 50 ml d'acétate d'éthyle contenant 1,17 g de NEM, on ajoute successivement 951 mg de H-Sta-OEt, 486 mg de HOBt et 1,71 g de Boc-Lys(Z)-ONSu. Agiter à TA, ajuster le pH à 6—7 par NEM, si nécessaire. Après 24 h, laver la solution organique successivement par KH $SO_4$—$K_2SO_4$, eau/ClNa, $NaHCO_3$ 5%, eau/ClNa. Sécher sur $MgSO_4$, évaporer le solvant à sec et triturer le résidu dans l'éther. Sécher après essorage.

Rendement: 1,17 g (75%)

F.: 90—93°C.

2. H-Lys(Z)-Sta-OEt, TFA (MB IV 563)

900 mg de Boc-Lys(Z)-Sta-OEt sont recouverts de 8 ml de TFA et TA. Après 15 min, évaporer le solvant, reprendre le résidu dans l'éther, évaporer l'éther: nous obtenons une huile qui est utilisée ainsi.

3. iVa-Phe-Lys(Z)-Sta-OEt

L'huile obtenue de H-Lys(Z)-Sta-OEt, TFA est solubilisée dans 50 ml de AcOEt. Ajouter 343 mg de HOBt puis NEM jusqu'à l'obtention de pH 7—8 au papier pH. Ajouter 623 mg de iVa-Phe-ONSu et agiter à TA. Ajuster le pH à 6—7 par NEM, si nécessaire. Après 24 h, essorer le solide, laver avec AcOEt et éther. Sécher.

Rendement: 910 mg (87%).

4. iVa-Phe-Lys(Z)-Sta-OH

869 mg de iVa-Phe-Lys(Z)-Sta-OEt sont solubilisés dans 30 ml de DMF. Ajouter 3 ml de soude normale. Agiter 30 min à TA, puis ajouter 3 ml d'acide chlorhydrique normal: le pH est alors de 6—7 au papier pH.

Evaporer les solvants sous haut vide au bain-marie à 40°C. Triturer le résidu dans l'eau, essorer le solide, laver à l'eau puis à l'éther. Sécher.

Rendement: 800 mg (100%).

5. iVa-Phe-Lys(Z)-Sta-Ala-Sta-OMe

432 mg de Boc-Ala-Sta-OMe sont recouverts de 5 ml de TFA à TA. Après 15 min, évaporer le solvant à fond; reprendre le résidu dans le DMF (85 ml) et amener à pH 7—8 par DIPEA, puis ajouter une solution de 800 mg de iVa-Phe-Lys(Z)-Sta-OH dans 20 ml de DMF contenant 155 mg de DIPEA, puis une solution de 645 mg. Agiter à TA durant 24 h. Evaporer le DMF contenant 186 mg de DIPEA. Agiter à TA durant 24 h. Evaporer le DMF sous haut vide, au bain-marie à 40°C. Reprendre le résidu dans l'eau, essorer le solide, laver à l'eau, laver à l'éther, avec AcOEt, puis à l'éther. Sécher.

Rendement: 950 mg (87%).

Spectre de RMN

| δ | Aspects | Intégration | Attribution | |
|---|---|---|---|---|
| 0,48—0,93 | m | 18 H | $^{6}C(CH_3)_2$ | Sta |
| | | | $^{3}C(CH_3)_2$ | iVa |
| 0,93—1,90 | m | 18 H | $CH_3$ | Ala |
| | | | $^{5}CH_2$—$^{6}CH$ | Sta |
| | | | $^{3}CH$—$^{2}CH_2$ | iVa |
| | | | $\beta\gamma\delta(CH_2)_3$ | Lys |
| 1,96—2,37 | m | 4 H | $^{2}CH_2$ | Sta |
| 2,58—3,04 | m | 4 H | $\varepsilon CH_2$ | Lys |
| | | | $^{3}CH_2$ | Phe |
| 3,50 | s | 3 H | $CH_3$ | $COOCH_3$ |
| 3,64—3,88 | m | 4 H | $^{4}CH$—$^{3}CH$ | Sta |
| 4,08—4,29 | m | 2 H | $^{2}CH$ | Ala |
| | | | $^{2}CH$ | Lys |
| 4,37—4,64 | m | 1 H | $^{2}CH$ | Phe |
| 4,72—5,04 | 2d+1S | 4 H | OH | Sta |
| | | | OH | |
| | | | $CH_2$ | Z |
| 7,03—7,45 | m | 13 H | 2 aromatiques | |
| | | | 3 NH | |
| 7,74—8,09 | 3 d | 3 H | 3 NH | |

Exemple 44

iVa-Phe-Phe-Sta-Ala-Sta-$NH_2$ (SR 42238)

1. H-Sta-OEt, TFA

5 g de Boc-Sta-OEt sont recouverts de 40 ml de TFA. L'ensemble s'échauffe légèrement. Après 20 min, évaporer le solvant à fond, reprendre l'huile résiduelle dans l'éther et le solide obtenu est essoré, lavé à l'éther et séché.

Rendement: 3,6 g (72%).

2. Boc-Phe-Sta-OEt

Dans 50 ml d'acétate d'éthyle contenant 1,56 g de NEM, on ajoute successivement 1,26 g de H-Sta-OEt, TFA, 648 mg de HOBt et 1,73 g de Boc-Phe-ONSu. Agiter à TA et ajuster le pH à 6—7, si nécessaire. Après une nuit, laver successivement la solution organique par $KHSO_4$—$K_2SO_4$ 5%, H2O/ClNa, $NaHCO_3$ 5%, H2O/ClNa. Sécher sur $MgSO_4$. Concentrer le solvant à quelques ml et le solide obtenu est essoré, lavé à l'éther et séché.

Rendement: 1,39 g (77%).

3. H-Phe-Sta-OEt, TFA

1 g de Boc-Phe-Sta-OEt sont recouverts de 8 ml de TFA. Après 20 min à TA, évaporer le solvant, ajouter de l'éther puis du pentane. Triturer afin de cristalliser l'huile, essorer le solide, laver au pentane, sécher sous vide.

Rendement: 1,01 g (100%).

4. iVa-Phe-Phe-Sta-OEt

Dans 20 ml de dioxane contenant 1,08 g de NEM, on ajoute successivement 1 g de H-Phe-Sta-OEt, TFA, 446 mg de HOBt et 1,15 g de iVa-Phe-ONSu. Agiter à TA; ajuster le pH à 6—7 par NEM, si nécessaire. Après une nuit, essorer le solide, laver le dioxane à l'éther. Sécher.

Rendement: 1,06 g (65%).

5. iVa-Phe-Phe-Sta-OH

1 g de iVa-Phe-Phe-Sta-OEt est solubilisé dans 300 ml de DMF. Ajouter 20 ml d'eau distillée puis à TA, 4 ml de soude normale. Agiter 30 min à TA puis ajouter 4 ml d'acide chlorhydrique normal: le pH est alors à 5. Evaporer les solvants sous haut vide au bain-marie à 40°C. Reprendre le résidue dans l'eau, essorer, rincer à l'éther. Sécher sous vide.

Rendement: 890 mg (93%).

6. Boc-Ala-Sta-$NH_2$ (MB IV 582)

500 mg de Boc-Ala-Sta-OMe sont solubilisés dans 20 ml de méthanol. Refroidir et ajouter 20 ml d'ammoniac liquide. Fermer la bombe et laisser au repos 4 jours à TA. Refroidir la bombe et, après ouverture, faire barboter de l'azote pour chasser l'ammoniac, puis évaporer le solvant. Reprendre le résidu dans un peu d'éther: nous obtenons un solide blanc; essorer. Sécher.

Rendement: 300 mg (65%)

F.: 175—178°C.

7. H-Ala-Sta-$NH_2$, TFA

300 mg de Boc-Ala-Sta-$NH_2$ sont recouverts de 3 ml de TFA à TA. Après 20 min, évaporer le solvant, reprendre le résidu dans l'éther, triturer, essorer le solide blanc, laver à l'éther. Sécher.

Rendement: 320 mg (103%).

8. iVa-Phe-Phe-Sta-Ala-Sta-$NH_2$

Dans 10 ml de dioxane contenant 124 mg de DIPEA, on ajoute successivement 107 mg de H-Ala-Sta-$NH_2$, TFA, 105 mg de iVa-Phe-Phe-Sta-OH et 162 mg de BOP. Agiter vigoureusement à TA; ajuster le pH à 6—7 par DIPEA, si nécessaire. Après une nuit, essorer le précipité, laver avec l'acétate d'éthyle l'éther. Sécher.

Rendement: 130 mg (58%)

Analyse d'acides aminés: Ala: 0,95 (1)
Sta: 2,04 (2)
Phe: 2,01 (2).

Spectre de RMN

| δ | Aspects | Intégration | Attribution | |
|---|---|---|---|---|
| 0,45—0,88 | m | 18 H | $^6C(CH_3)_2$ | Sta |
| | | | $^3C(CH_3)_2$ | iVa |
| 1,06—2,12 | m | 16 H | $^5CH_2—^6CH$ | Sta |
| | | | $CH_3$ | Ala |
| | | | $^2CH_2—^3CH$ | iVa |
| | | | $^2CH_2$ | Sta |
| 2,51—3,06 | m | 4 H | $^2CH_2$ | Phe |
| 3,61—4,59 | m | 7 H | $^4CH—^3CH$ | Sta |
| | | | $^2CH$ | Phe |
| | | | $^2CH$ | Ala |
| 4,79—4,88 | m | 2 H | OH | Sta |
| | | | OH | |
| 6,75 | s | 1 H | NH | $NH_2$ |
| 7,00—7,27 | m | 11 H | 2 aromatiques | Phe |
| | | | NH | $PH_2$ |
| 7,35 | d | 1 H | NH | |
| 7,5 | d | 1 H | NH | |
| 7,8 | d | 1 H | NH | |
| 7,88 | d | 1 H | NH | |
| 8,08 | d | 1 H | NH | |

Exemple 45

t-Bu-Ac-Phe-Phe-Sta-Ala-Sta-OMe (SR 42256)

Dans 10 ml de dioxane, on ajoute successivement 114 mg de NEM, 200 mg de H-Phe-Sta-Ala-Sta-OMe, TFA (exemple 1—6), 126 mg de t-BuAc-Phe-ONSu et 47 mg de HOBt. Agiter à TA; ajuster le pH à 6—7 par NEM, si nécessaire. Après 24 h, évaporer le dioxane, reprendre le résidu dans AcOEt et essorer le solide. Laver avec AcOEt puis à l'éther. Sécher.

Rendement: 170 mg (72%).

Spectre de RMN

| δ | Aspects | Intégration | Attribution | |
|---|---|---|---|---|
| 0,66—0,95 | m | 21 H | $^{6}C(CH_3)_2$ | Sta |
| | | | $^{3}C(CH_3)_3$ | tBuAc |
| 1,08—1,61 | m | 9 H | $^{5}CH_2$—$^{6}CH$ | Sta |
| | | | $CH_3$ | Ala |
| 1,82 | s | 2 H | $^{2}CH_2$ | tBuAc |
| 1,88—2,40 | m | 4 H | $^{2}CH_2$ | Sta |
| 2,48—3,24 | m | 4 H | $^{3}CH_2$ | Phe |
| 3,51 | s | 3 H | $CH_3$ | $COOCH_3$ |
| 3,69—4,59 | m | 7 H | $^{4}CH$—$^{3}CH$ | Sta |
| | | | $^{2}CH$ | Phe |
| | | | $^{2}CH$ | Ala |
| 4,8 | d | 1 H | OH | Sta |
| 4,96 | d | 1 H | OH | |
| 7,03—7,24 | m | 10 H | 2 aromatiques | |
| 7,39 | d | 1 H | NH | |
| 7,46 | d | 1 H | NH | |
| 7,72—7,88 | m | 2 H | 2 NH | |
| 8,08 | d | 1 H | NH | |

Exemple 46
iVa-Phe-Ala-Sta-Ala-Sta-OMe (SR 42258)

1. iVa-Phe-Ala-Sta-OMe

700 mg de Boc-Al-Sta-OMe sont recouverts de 5 ml de TFA. Après 15 min à TA, évaporer le solvant à fond et dissoudre l'huile résiduelle dans 20 ml de dioxane; amener à pH 8 par NEM, au papier pH, puis ajouter 297 mg de HOBt et 761 mg de iVa-Phe-ONSu. Agiter à TA; ajuster le pH à 6—7 par NEM, si nécessaire. Après 24 h, essorer le précipité, laver celui-ci avec AcOEt et à l'éther. Sécher.

Rendement: 570 mg (76%)

2. iVa-Phe-Ala-Sta-OH

491 mg de iVa-Phe-Ala-Sta-OMe sont solubilisés dans 20 ml de DMF à TA. Ajouter 2 ml de soude normale et agiter 40 min à TA puis ajouter 2 ml d'acide chlorhydrique normal: le pH est à 5—6 au papier pH. Evaporer les solvants sous haut vide, reprendre le résidu dans l'eau; essorer le solide, laver celui-ci à l'eau. Dissoudre dans MeOH et évaporer à fond. Reprendre de nouveau dans le minimum de MeOH et précipiter le produit par addition d'éther. Sécher.

Rendement: 380 mg (79%)

3. iVa-Phe-Ala-Sta-Ala-Sta-OMe

180 mg de Boc-Ala-Sta-OMe sont recouverts de 2 ml de TFA à TA. Après 15 min à TA, évaporer le solvant et dissoudre le résidu dans 10 ml de dioxane; amener à pH 8 par DIPEA et ajouter 238 mg de iVa-Phe-Ala-Sta-OH et 269 mg de BOP. Agiter à TA; ajuster le pH à 6—7 par DIPEA, si nécessaire. Après 24 h, essorer le solide, laver avec AcOEt et éther. Sécher.

Rendement: 320 mg (88%)

Spectre de RMN

| δ | Aspects | Intégration | Attribution | |
|---|---|---|---|---|
| 0,56—0,88 | m | 18 H | $^6C(CH_3)_2$ | Sta |
| | | | $^3C(CH_3)_2$ | Iva |
| 1,08—1,91 | m | 15 H (·) | $^2CH_3$ | Ala |
| | | | $^5CH_2—^6CH$ | Sta |
| | | | $^2CH_2—^3CH$ | iVa |
| 1,95—2,38 | m | 4 H | $^2CH_2$ | Sta |
| 2,59—3,17 | m | 2 H | $^2CH_2$ | Phe |
| 3,51 | s | 3 H | $CH_3$ | $COOCH_3$ |
| 3,66—3,90 | m | 4 H | $^4CH—^3CH$ | Sta |
| 4,06—4,35 | m | 2 H | $^2CH$ | Ala |
| 4,40—4,56 | m | 1 H | $^2CH$ | Phe |
| 4,82 | d | 1 H | OH | Sta |
| 4,95 | d | 1 H | OH | |
| 7,04—7,25 | m | 5 H | aromatique | |
| 7,29—7,41 | m | 2 H | 2 NH | |
| 7,90 | d | 1 H | NH | |
| 8,00 | d | 1 H | NH | |
| 8,08 | d | 1 H | NH | |
| (·) Excès d'intégration | | | | |

Exemple 47

iVa-Phe-Gly-Sta-Ala-Sta-OMe (SR 42261)

1. iVa-Phe-Gly-Sta-OMe

280 mg de Boc-Gly-Sta-OMe sont recouverts de 3 ml de TFA à TA. Après 15 min, évaporer le solvant à fond. Reprendre le résidu dans 10 ml de dioxane et amener à pH 8 par NEM. Ajouter 118 mg de HOBt et 304 mg de iVa-Phe-ONSu. Agiter à TA et ajuster le pH à 6—7 par NEM, si nécessaire. Après 24 h, évaporer le dioxane, dissoudre le résidu dans AcOEt et laver successivement par $KHSO_4$—$K_2SO_4$ 5%, eau/ClNa, $NaHCO_3$ 5%, eau/ClNa. Sécher sur $MgSO_4$. Evaporer le solvant, gratter le résidu dans le pentane, essorer, sécher.

Rendement: 270 mg (70%)

2. iVa-Phe-Gly-Sta-OH

238 mg de iVa-Phe-Gly-Sta-OMe sont solubilisés dans 10 ml de dioxane et 2 ml de MeOH à TA. Ajouter 1 ml de soude normale et agiter à TA durant 1 h. Ajouter 1 ml d'acide chlorhydrique. Evaporer les solvants à sec, reprendre le résidu dan un peu d'eau, triturer, décanter, reprendre dans MeOH et évaporer à sec. Triturer dans l'éther, essorer. Sécher.

Rendement: 190 mg (82%)

3. iVa-Phe-Gly-Sta-Ala-Sta-OMe

144 mg de Boc-Ala-Sta-OMe sont recouverts de 2 ml de TFA à TA. Après 15 min, évaporer le solvant à fond, dissoudre le résidu dans 10 ml de dioxane et amener à pH 7—8 par DIPEA puis ajouter 185 mg de iVa-Phe-Gly-Sta-OH et 215 mg de BOP. Agiter à TA, ajuster le pH à 6—7 par DIPEA, is nécessaire. Après 24

h, filtrer, évaporer le solvant, dissoudre le résidu dans AcOEt et laver la solution organique successivement par $KHSO_4$—$K_2SO_4$ 5%, eau-ClNa, $NaHCO_3$ 5%, eau-ClNa. Sécher sur $MgSO_4$, évaporer le solvant. Dissoudre le produit dans le chloroform et déposer en tête d'une colonne (L: 45 cm, diamètre: 1,5 cm) de gel de silice 60 Merck (70—230 mesh) dans le chloroforme. Eluer avec un gradient MeOH-chloroforme de 1—99 à 5—95. Fractionner. Evaporer les fractions contenant le produit pur. Reprendre le résidu dans le pentane, essorer. Sécher.

Rendement: 120 mg (42%).

Spectre de RMN

| δ | Aspects | Intégration | Attribution | |
|---|---|---|---|---|
| 0,51—0,87 | m | 18 H | $^6C(CH_3)_2$ | Sta |
| | | | $^3C(CH_3)_2$ | iVa |
| 1,04—1,88 | m | 12 H | $CH_3$ | Ala |
| | | | $^5CH_2$—$^6CH$ | Sta |
| | | | $^2CH_2$—$^3CH$ | iVa |
| 1,95—2,37 | m | 4 H | $^2CH_2$ | Sta |
| 2,56—3,00 | m | 2 H | $^2CH_2$ | Phe |
| 3,40—3,88 | m | (·) | $CH_3$ | $COOCH_3$ |
| | | | $CH_2$ | Gly |
| | | | $^4CH$—$^3CH$ | Sta |
| 4,08—4,24 | m | 1 H | $^2CH$ | Ala |
| 4,32—4,50 | m | 1 H | $^2CH$ | Phe |
| 4,79 | d | 1 H | OH | Sta |
| 4,90 | d | 1 H | OH | |
| 7,00—7,20 | m | 5 H | aromatique | |
| 7,20—7,38 | m | 2 H | 2 NH | |
| 7,83 | d | 1 H | NH | |
| 8,04 | d | 1 H | NH | |
| 8,20 | m | 1 H | NH | |

(·) Déficit d'intégration dû à l'irradiation du pic de l'eau

Les produits selon l'invention ont été étudiés en ce qui concerne leurs propriétés thérapeutiques et notamment leur action inhibitrice enzymatique. Plus particulièrement, les composés ont été évalués "in vitro", d'une part, sur l'inhibition de l'activité Rénine Plasmatique Humaine et, d'autre part, sur l'inhibition de la Pepsine de Porc.

Quelques produits ont également été traités sur l'inhibition de l'activité de la Cathépsine D.

I. Methodes

1. Inhibition de l'activite renine plasmatique humaine (A.R.P.)

Notre méthode est inspirée de Guyene (J. Clin. Endocri. Metab. *43*, p. 1301, 1976) dans la mesure où l'inhibition de l'A.R.P. est évaluée à partir d'un pool de plasmas humains, riche en rénine (15 à 20 ng d'antiotensine I libérée par millilitre et par heure) incubé à 37°C en présence de concentrations croissantes du produit à étudier.

L'angiotensine I libérée au cours de la réaction (les plasmas humains contiennent et le subtrat: l'angiotensinogène, et l'enzyme: la rénine) est mesurée par dosage radio-immunologie à l'aide d'un kit.

Bien entendu, un inhibiteur de l'enzyme de conversion du PMSF (phénylméthylsulfonyl)fluorure est rajouté au milieu d'incubation.

Deux types d'expériences ont été effectués:

— l'un à pH 6 qui est le pH optimal de la réaction enzymatique avec la rénine humaine (tampon maléate, incubation 30 min),
— l'autre à pH 7,4 qui est le pH physiologique (tampon phosphate, incubation 60 min).

Les résultats sont exprimés par la dose de composé évaluée en moles qui inhibe de 50% (IC50) l'Activité Rénine Plasmatique Humaine présente en l'absence d'inhibiteur.

2. Inhibition de la pepsine de porc

La méthode utilisée est celle de Takaaki Aoyagi (J. Antibiotics, 24, p. 687—694, 1971). Le substrat utilisé est de l'hémoglobine bovine, l'enzyme est de la pepsine de porc.

L'hémoglobine à 0,5% est hydrolysée par la pepsine (1 mcg/ml) à 37°C dans du tampon KCl—HCl 0,02 M à pH 2.

Une pré-incubation de 3 min a lieu, suivie d'une incubation de 25 min. Après précipitation des protéines à l'acide perchlorique 1,7 M, on détermine la densité optique du surnageant au spectrophotomètre à 280 nm.

Les résultats sont exprimés en pourcentages d'inhibition de la densité optique maximale obtenue en absence d'inhibiteur. Ils sont également exprimés par une IC 50.

3. Inhibition enzymatique de la cathepsine D

La méthode utilisée est très voisine de celle de la pepsine (T. Aoyagi, 1971).

Le substrat utilisé est de hémoglobine bovine, l'enzyme est de la cathépsine D d'origine bovine (Sigma, n° C 3138, Lot 26 C-8100).

L'hémoglobine à 0,5% est hydrolysée par la cathépsine D (200 µg/ml) à 37°C dans du tampon citrate-phosphate à pH 3,2.

Le substrat, l'inhibiteur et l'enzyme sont mis à incuber pendant 30 min.

Après précipitation des protéines à l'acide perchlorique 1,7 M, on détermine la densité optique du surnageant au spectrophotomètre à 280 nm.

Les résultats sont exprimés en pourcentage de diminution de la densite optique maximale obtenue en absence d'inhibiteur. Le tableau des résultats rassemble les concentrations qui inhibent 50% de l'effet maximal (IC50).

4. Solvants des peptides utilises pour les trois methodes

Une solution mère du peptide à $10^{-3}$ M est effecutée dans un mélange contenant volume à volume une solution A (19 ml méthanol+1 ml acide acétique) et une solution B (4 ml méthanol+2 ml soude).

Les dilutions ultérieures du peptide sont alors effectuées dans le tampon adéquat selon les protocoles décrits ci-dessus.

La quantité de solvant présente dans une solution du peptide à une concentration inférieure à $10^{-4}$ n'interfère pas avec les résultats.

II. Résultats

Les résultats obtenus avec divers produits de l'invention sont représentés dans les tableaux suivants où figurent les IC50 de chaque molécule en ce qui concerne leur inhibition de l'activité rénine plasmatique humaine, leur inhibition de la pepsine et leur inhibition de la cathépsine D. De 5 à 10 doses ont été nécessaires pour déterminer ces IC50. La pepstatine, en tant que substance de référence, est toujours testée en parallèle dans chaque expérience.

TABLEAU I

| N° code | Inhibition A.R.P.△ humaine (IC50)■ | | Inhibition Pepsine porcine (IC50) pH 2 |
|---|---|---|---|
| | pH 6 | pH 7,4 | |
| Pepstatine | 1,2 à 1,6×$10^{-}$M | 1,2 à 1,4×$10^{-5}$M | 1,5 à 2,8×$10^{-8}$M |
| SR 41225 | 7×$10^{-8}$M | $10^{-6}$M | 9×$10^{-8}$M |
| SR 41320 | 1,7×$10^{-8}$M | 3,5×$10^{-6}$M | 3×$10^{-8}$M |
| SR 41331 | 3,5×$10^{-9}$M | 1,9×$10^{-7}$M | 1,4×$10^{-8}$M |
| SR 41376 | 1,7×$10^{-8}$M | 1,5×$10^{-7}$M | 1,2×$10^{-7}$M |
| SR 41377 | 3×$10^{-7}$M | $10^{-5}$M | 4,7×$10^{-8}$M |
| SR 41395 | 1,8×$10^{-9}$M | 2,7×$10^{-8}$M | 1,3×$10^{-6}$M |
| SR 41405 | $10^{-8}$M | 1,8×$10^{-7}$M | 6,5×$10^{-8}$M |
| SR 41416 | 8×$10^{-8}$M | 1,4×$10^{-6}$M | 6,5×$10^{-8}$M |
| SR 41476 | 1,7×$10^{-7}$M | 2,2×$10^{-6}$M | 2,4×$10^{-8}$M |
| SR 41477 | 3,5×$10^{-8}$M | $10^{-6}$M | 3,8×$10^{-8}$M |
| SR 41485 | 6,75×$10^{-9}$M | 1,5×$10^{-7}$M | 2,9×$10^{-8}$M |
| SR 41491 | 2,6×$10^{-7}$M | 4×$10^{-6}$M | 2,2×$10^{-8}$M |
| SR 41492 | 4×$10^{-9}$M | 4,5×$10^{-8}$M | 5,5×$10^{-7}$M |
| SR 41518 | 9,5×$10^{-7}$M | — | 4,8×$10^{-7}$M |

TABLEAU 1 (suite 1)

| N° code | Inhibition A.R.P. Humaine (IC50) | | Inhibition pepsine porcine (IC50) pH 2 |
|---|---|---|---|
| | pH 6 | pH 7,4 | |
| SR 41748 | $2,2\times10^{-9}$ | $1,1\times10^{-7}$ | $6,2\times10^{-7}$ |
| SR 41764 | $1,2\times10^{-8}$ | $3,2\times10^{-7}$ | $4,2\times10^{-8}$ |
| SR 41765 | $3,3\times10^{-10}$ | $7,5\times10^{-8}$ | $8,0\times10^{-9}$ |
| SR 41766 | $1,4\times10^{-8}$ | $4,2\times10^{-7}$ | $5,8\times10^{-8}$ |
| SR 41768 | $5,0\times10^{-8}$ | $1,0\times10^{-6}$ | $4,2\times10^{-8}$ |
| SR 41919 | $2,0\times10^{-11}$ | $1,1\times10^{-7}$ | $2,0\times10^{-8}$ |
| SR 41938 | $1,6\times10^{-9}$ | $4,4\times10^{-7}$ | $6,3\times10^{-8}$ |
| SR 41939 | $1,7\times10^{-8}$ | $8,8\times10^{-7}$ | $3,6\times10^{-8}$ |
| SR 41994 | $7,6\times10^{-10}$ | $2,6\times10^{-7}$ | $1,3\times10^{-8}$ |
| SR 41995 | $3,0\times10^{-11}$ | $4,2\times10^{-8}$ | $1,6\times10^{-8}$ |
| SR 41996 | $1,1\times10^{-12}$ | $5,1\times10^{-8}$ | $1,4\times10^{-8}$ |
| SR 42019 | $1,1\times10^{-7}$ | $3,5\times10^{-7}$ | $7,3\times10^{-8}$ |
| SR 42062 | $6,1\times10^{-8}$ | $1,7\times10^{-6}$ | $2,6\times10^{-8}$ |

TABLEAU 1 (suite 2)

| N° code | Inhibition A.R.P. Humaine (IC50) | | Inhibition pepsine porcine (IC50) pH 2 |
|---|---|---|---|
| | pH 6 | pH 7,4 | |
| SR 42128 | $2,4\times10^{-13}$ | $2,8\times10^{-8}$ | $1,9\times10^{-8}$ |
| SR 42129 | $4,7\times10^{-10}$ | $3,0\times10^{-7}$ | $1,6\times10^{-8}$ |
| SR 42130 | $5,2\times10^{-8}$ | $2,6\times10^{-6}$ | $2,7\times10^{-8}$ |
| SR 42238 | $2,1\times10^{-9}$ | $1,9\times10^{-7}$ | $2,1\times10^{-8}$ |
| SR 42256 | $2,6\times10^{-8}$ | $4,8\times10^{-7}$ | $2,7\times10^{-8}$ |
| SR 42258 | $9,0\times10^{-9}$ | $4,7\times10^{-7}$ | $1,8\times10^{-8}$ |
| SR 42261 | $1,7\times10^{-7}$ | $4,7\times10^{-6}$ | $7,5\times10^{-7}$ |
| SR 42286 | $1,2\times10^{-6}$ | $1,1\times10^{-5}$ | $>10^{-5}$ |

△ A.R.P.=Activite Renine plasmatique

■ IC50=Dose inhibitrice de 50% de l'effet servant de référence.

TABLEAU 2
Inhibition de l'activite enzymatique de la cathepsine D

| N° code | Formules | IC50 |
|---|---|---|
| SR 41331 | Boc-Phe-Phe-Sta-Ala-Sta-OMe | $1,8\times10^{-7}$M |
| SR 41376 | Boc-Trp-Trp-Sta-Al-Sta-OMe | $1,3\times10^{-7}$M |
| SR 41395 | Boc-Phe-His-Sta-Ala-Sta-OMe | $8,7\times10^{-7}$M |
| SR 41405 | Z-Phe-Val-Sta-Ala-Sta-OMe | $9,5\times10^{-8}$M |
| SR 41485 | Z-Trp-Val-Sta-Ala-Sta-OMe | $7,5\times10^{-8}$M |
| SR 41492 | Boc-Phe-His-Sta-Ala-Sta-OH | $7,0\times10^{-7}$M |
| SR 41619 | Boc-Trp-His-Sta-Ala-Sta-OMe | $7,5\times10^{-7}$M |
| Pepstatine | Iv-Val-Val-Sta-Ala-Sta | $6,2\times10^{-8}$M |

Les composés de la présente invention ont une action inhibitrice sur l'activité rénine plasmatique humaine très important et de façon générale nettement supérieure à celle du produit naturel: la pepstatine, à ce titre ils peuvent être utilisés dans le traitement de l'hypertension artérielle.

Ils possèdent également une action inhibitrice marquée sur les protéines acides, notamment la pepsine et la cathépsine D. On peut donc envisager l'utilisation des produits selon l'invention dans les domaines thérapeutiques où l'inhibition de tels systèmes enzymatiques est justifiée, notamment outre l'hypertension artérielle, l'ulcère gastroduodénal et les affections inflammatoires.

Les peptides de la présente invention peuvent être utilisés en thérapeutique par voie injectable: intraveineuse, intramusculaire ou sous-cutanée. Ils sont utilisés dans un solvant tel que le sérum physiologique (solution saline isotonique) ou dans un tampon tel que le tampon phosphate.

La quantité de principe actif à utiliser varie suivant les effets thérapeutiques recherchés, la gravité de l'affection à traiter et la voie d'administration choisie. Elle doit être déterminée pour chaque patient et est le plus souvent comprise entre 1 et 100 mg de principe actif.

**Revendications pour les Etats Contractants: BE, CH, DE, FR, GB, IT, LI, LU, NL, SE**

1. Dérivés peptidiques de formule générale

$$R—X—Y—Statyl_1—Ala—Statyl—R' \qquad (I)$$

dans laquelle
— $Statyl_1$ représente le radical, décrivant de l'aminoacide statine, de formule

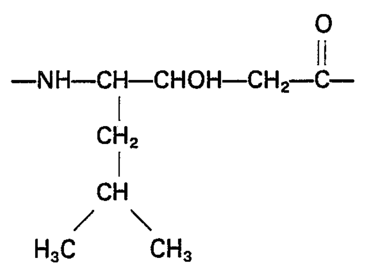

isomère 3S, 4S

et $Statyl_2$ le radical statyl dérivant du même aminoacide qu'il s'agisse de l'isomère 3S, 4S ou de l'isomère 3R, 4S.
— R désigne un atome d'hydrogène ou un groupement acylant fixé sur le groupe amino terminal de l'aminoacide X,
— X et Y identiques ou différents désignent des acides aminés de configuration L ou D lorsqu'il existe un centre d'asymétrie dans la molécule, et éventuellement protégés dans leur chaîne latérale, lesdits acides aminés étant choisis parmi les acides aminés suivants:
— X: Phénylalanine, Tryptophane, Histidine, Boc Histidine, Tyrosine, Proline, Isoleucine;
— Y: Phénylalanine, Tryptophane, Histidine, Tyrosine, Proline, Leucine, Isoleucine, Valine, Glycine;
— R': représente OH, O-alkyle inférieur,

et les sels, pharmaceutiquement acceptables des produits de formule (I) lorsque R' représente OH.

2. Dérivés peptidiques selon la revendication 1, dans lesquels R représente un groupement choisi parmi les groupements acétyl (Ac), isovaléryl (i, Va), octyl, t. butylacétyl, t.butoxy-carbonyl (Boc), adamantyloxycarbonyl (A doc), benzyloxycarbonyl (Z), (benzyloxycarbonyl) β-aminoéthylsulfonyl (Z-Tau), (t.butyloxy-carbonyl) β-amino-éthylsulfonyl (Boc-Tau), phénylsulfonyl, benzylsulfonyl, phényl-3 propionyl.

3. Procédé de préparation des produits de formule (I), caractérisé en ce que l'on utilise comme produit de départ un ester d'alkyl inférieur de la statine à partir duquel on couple, étape par étape, les divers acides aminés convenablement protégés, le produit obtenu à chaque étape étant déprotégé, selon des procédés connus, avant d'être soumis à nouveau couplage, chacune des opérations de couplage étant effecutée en utilisant, soit un ester activé de l'amino-acide à coupler, soit l'amino-acide N protégé en présence de dicyclohexyl-carbodiimide.

4. Procédé selon la revendication 3, caractérisé en ce que la déprotection du produit obtenu est effectuée par hydrogénolyse ou par hydrolyse en milieu acide fort.

5. Procédé selon l'une des revendications 3 et 4, caractérisé en ce qu'en fin de réaction la fonction ester d'alkyl inférieur de la statine est saponifiée en milieu alcalin, de façon à obtenir les produits de formule (I) dans lesquels R' est H.

6. Médicaments caractérisés en ce qu'ils contiennent un produit de formule (I).

7. Medicaments selon la revendication 6, utilisables notamment pour le traitement de la tension artérielle et de maladies digestives, caractérisés en ce qu'ils contiennent de 1 à 100 mg de produit de formule (I).

**Revendications pour l'Etat Contractant: AT**

1. Procédé de préparation de dérivés peptidiques de formule générale

$$R—X—Y—Statyl_1—Ala—Statyl_2—R' \quad (I)$$

dans laquelle

— $statyl_1$ représente le radical, dérivant de l'aminoacide statine, de formule

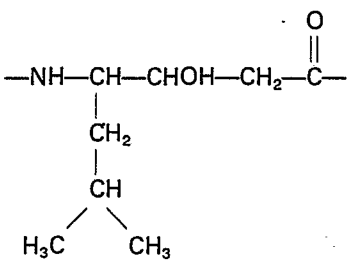

isomère 3 S, 4 S

et $Statyl_2$ le radical statyl dérivant du même amino-acide qu'ils s'agisse de l'isomère 3S, 4S ou de l'isomère 3R, 4S,

— R désigne un atome d'hydrogène ou un groupement acylant fixé sur le groupe amino terminal de l'aminoacide X,

— X et Y identiques ou différents désignent des acides aminés de configuration L ou D lorsqu'il existe un centre d'asymétrie dans la molécule, et éventuellement protégés dans leur chaîne latérale,

lesdits acides aminés étant choisis parmi les acides aminés suivants:

— X: Phénylalanine, Tryptophane, Histidine, Boc Histidine, Tyrosine, Proline, Isoleucine;

— Y: Phénylalanine, Tryptophane, Histidine, Tyrosine, Proline, Leucine, Isoleucine, Valine, Glycine;

— R': représente OH, O-alkyle inféreiur,

et les sels pharmaceutiquement acceptables des produits de formule I), lorsque R' représente OH,

caractérisé en ce que l'on utilise comme produit de départ un ester d'alkyl inférieur de la statine à partir duquel on couple, étape par étape, les divers acides aminés convenablement protégés, le produit obtenu à chaque étape étant déprotégé, selon des procédés connus, avant d'être soumis à un nouveau couplage, chacune des opérations de couplage étant effectuée en utilisant, soit un ester activé de l'aminoacide à coupler, soit l'amino-acide N protégé en présence de dicyclohexyl-carbodiimide.

2. Procédé selon la revendication 1, caractérisé en ce que la déprotection du produit obtenu est effectué par hydrogènolyse ou par hydrolyse en milieu acide fort.

3. Procédé selon l'une des revendications 1 et 2 caractérisé en ce qu'en fin de réaction la fonction ester d'alkyl inférieur de la statine est saponifée en milieu alcalin, de façon à obtenir les produits de formule (1) dans lesquels R' est H.

**Patentansprüche für die Vertragsstaaten: BE, CH, DE, FR, GB, IT, LI, LU, NL, SE**

1. Peptidderivate der allgemeinen Formel

$$R—X—Y—Statyl_1—Ala—Statyl_2—R' \quad (I)$$

worin

— $Statyl_1$ die von der Aminosäure Statin abgeleitete Gruppe der Formel

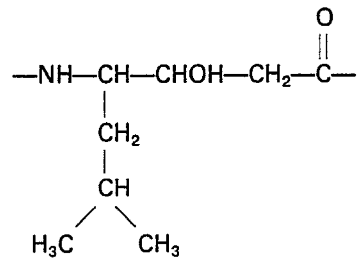

3S,4S-Isomeres

darstellt und $Statyl_2$ die von derselben Aminosäure abgeleitete Statylgruppe bedeutet, jenachdem, ob es sich um das 3S,4S-Isomere oder das 3R,4S-Isomere handelt,

— R ein Wasserstoffatom oder eine an der terminalen Aminogruppe der Aminosäure X hängende acylierende Gruppe bezeichnet,

— X und Y, gleich oder verschieden, Aminosäuren der Konfiguration L oder D darstellen, wenn im Molekül ein Assymmetriezentrum besteht, die gegebenenfalls in ihrer Seitengruppe geschützt sind, welche Aminosäuren ausgewählt sind aus den folgenden Aminosäuren:

— X: Phenylalanin, Tryptophan, Histidin, Boc Histidin, Tyrosin, Prolin, Isoleucin;

— Y: Phenylalanin, Tryptophan, Histidin, Tyrosin, Prolin, Leucin, Isoleucin, Valin, Glycin;

— R' für OH, O-Niedrigalkyl steht,

und die pharmazeutisch akzeptablen Salze der Produkte der Formel (I), wenn R' für OH steht.

2. Peptidderivate nach Anspruch 1, bei welchen R eine Gruppe, ausgewählt aus den Gruppen Acetyl (Ac), Isovaleryl (i, Va), Octyl, tert. Butylacetyl, tert.Butoxycarbonyl (Boc), Adamantyloxycarbonyl (A doc), Benzyloxycarbonyl (Z), (Benzyloxycarbonyl)-β-aminoäthylsulfonyl (Z-Tau), (tert. Butyloxycarbonyl)-β-aminoäthylsulfonyl (Boc-Tau), Phenylsulfonyl, Benzylsulfonyl, 3-Phenylpropionyl, darstellt.

3. Verfahren zur Herstellung der Produkte der Formel (I), dadurch gekennzeichnet, daß man als Ausgangsprodukt einen Niedrigalkylester von Statin verwendet, ausgehend von welchem man Schritt für Schritt die verschiedenen entsprechend geschützten Aminosäuren ankoppelt, wobei das bei jedem Schritt erhaltene Produkt nach bekannten Verfahren entschützt wird, bevor es einer neuerlichen Kopplung unterzogen wird, wobei jeder der Kopplungsvorgänge unter Verwendung entweder eines aktivierten Esters der zu koppelnden Aminosäure oder der N-geschützten Aminosäure in Gegenwart von Dicyclohexylcarbodiimid durchgeführt wird.

4. Verfahren nach Anspruch 3, dadurch gekennzeichnet, daß die Entfernung der Schutzgruppe vom erhaltenen Produkt mittels Hydrogenolyse oder mittels Hydrolyse in stark saurem Milieu durchgeführt wird.

5. Verfahren nach einem der Ansprüche 3 und 4, dadurch gekennzeichnet, daß am Ende der Reaktion die Niedrigalkylesterfunktion des Statins in alkalischem Milieu verseift wird, sodaß die Produkte der Formel (I), worin R' für H steht, erhalten werden.

6. Medikamente, dadurch gekennzeichnet, daß sie ein Produkt der Formel (I) enthalten.

7. Medikamente nach Anspruch 6 zu verwenden insbesondere zur Behandlung des arteriellen Drucks und von Verdauungserkrankungen, dadurch gekennzeichnet, daß sie 1 bis 100 mg des Produkts der Formel (I) enthalten.

**Patentansprüche für den Vertragsstaat: AT**

1. Verfahren zur Herstellung von Peptidderivaten der allgemeinen Formel

$$R—X—Y—Statyl_1—Ala—Statyl_2—R' \quad (I)$$

worin

— $Statyl_1$ die von der Aminosäure Statin abgeleitete Gruppe der Formel

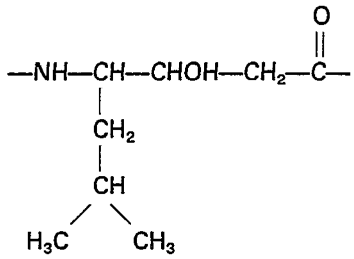

3S,4S-Isomeres

darstellt und $Statyl_2$ die von derselben Aminosäure abgeleitete Statylgruppe bedeutet, jenachdem, ob es sich um das 3S,4S-Isomere oder das 3R,4S-Isomere handelt,

— R ein Wasserstoffatom oder eine an der terminalen Aminogruppe der Aminosäure X hängende acylierende Gruppe bezeichnet,

— X und Y, gleich oder verschieden, Aminosäuren der Konfiguration L oder D darstellen, wenn in Molekül ein Assymmetriezentrum besteht, die gegebenenfalls in ihrer Seitengruppe geschützt sind, welche Aminosäuren ausgewählt sind aus den folgenden Aminosäuren:

— X: Phenylalanin, Tryptophan, Histidin, Boc Histidin, Tyrosin, Prolin, Isoleucin;
— Y: Phenylalanin, Tryptophan, Histidin, Tyrosin, Prolin, Leucin, Isoleucin, Valin, Glycin;
— R′ für OH, O-Niedrigalkyl steht,

und der pharmazeutisch akzeptablen Salze der Produkte der Formel (I), wenn R′ für OH steht, dadurch gekennzeichnet, daß man als Ausgangsprodukt einen Niedrigalkylester von Statin verwendet, ausgehend von welchem man Schritt für Schritt die verschiedenen entsprechend geschützten Aminosäuren ankoppelt, wobei das bei jedem Schritt erhaltene Produkt nach bekannten Verfahren entschützt wird, bevor es einer neuerlichen Kopplung unterzogen wird, wobei jeder der Kopplungsvorgänge unter Verwendung entweder eines aktivierten Esters de zu koppelnden Aminosäure oder der N-geschützten Aminosäure in Gegenwart von Dicyclohexylcarbodiimid durchgeführt wird.

2. Verfahren nach Anspruch 1, dadurch gekennzeichnet, daß die Entfernung der Schutzgruppe vom erhaltenen Produkt mittels Hydrogenolyse oder mittels Hydrolyse in stark saurem Milieu durchgeführt wird.

3. Verfahren nach einem der Ansprüche 1 und 2, dadurch gekennzeichnet, daß am Ende der Reaktion die Niedrigalkylesterfunktion des Statins in alkalischem Milieu verseift wird, sodaß die Produkte der Formel (I), worin R′ für H steht, erhalten werden.

**Claims for the Contracting States: BE, CH, DE, FR, GB, IT, LI, LU, NL, SE**

1. Peptide derivatives of the general formula:

$$R—X—Y—Statyl_1—Ala—Statyl_2—R' \qquad (I)$$

in which:

— $Statyl_1$ represents the radical derived from the aminoacid statine, of the formula

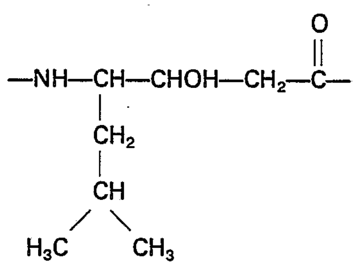

3S,4S isomer

and $Statyl_2$ denotes the statyl radical derived from the same aminoacid, but it can be the 3S,4S isomer or the 3R,4S isomer,

— R denotes a hydrogen atom or an acylating group attached to the terminal amino group of the aminoacid X,

— X and Y, which are identical or different, denote aminoacids of the L or D configuration when a centre of asymmetry exists in the molecule, and optionally protected in their side chain, said aminoacids being selected from the following aminoacids;

— X=Phenylalanine, Tryptophan, Histidine, Boc-Histidine, Tyrosine, Proline, Isoleucine;
— Y=Phenylalanine, Tryptophan, Histidine, Tyrosine, Proline, Leucine, Isoleucine, Valine, Glycine;
— R′ represents OH, O-lower alkyl, and the pharmaceutically acceptable salts of the products of the formula (I) when R′ represents OH.

2. Peptide derivatives according to claim 1, in which R represents a group selected from the following groups: acetyl (Ac), isovaleryl (iva), octyl, t-butylacetyl, t-butoxycarbonyl (Boc), adamantyloxycarbonyl (Adoc), benzyloxycarbonyl (Z), (benzyloxycarbonyl)-β-aminoethylsulphonyl (Z-Tau), (t-butyloxy-carbonyl)-β-aminoethylsulphonyl (Boc-Tau), phenylsulphonyl, benzylsulphonyl and 3-phenyl-propionyl.

3. Process for the preparation of the products of the formula (I), characterized in that the starting material used is a lower alkyl ester of statine, from which the various aminoacids, suitably protected are stepwise coupled the product obtained at each step being freed, in accordance with known processes, before being subjected to a further coupling, and each of the coupling operations being carried out using either an activated ester of the aminoacid to be coupled, or the N-protected aminoacid in the presence of dicyclohexylcarbodiimide.

4. Process according to claim 3, characterized in that the freeing of the product obtained is carried out by hydrogenolysis or by hydrolysis in a strong acid medium.

5. Process according to one of claims 3 and 4, characterized in that, at the end of the reaction, the lower alkyl ester group of the statine is saponified in an alkaline medium so as to give the products of the formula (I) in which R' is H.

6. Medicaments, characterized in that they contain a product of the formula (I).

7. Medicaments according to claim 6, which can be used in particular for the treatment of arterial pressure and digestive diseases, characterized in that they contain from 1 to 100 mg of product of the formula (I).

**Claims for the Contracting State: AT**

1. Process for preparing peptide derivatives of the general formula:

$$R—X—Y—Statyl_1—Ala—Statyl_2—R' \quad (I)$$

in which:

— $Statyl_1$ represents the radical derived from the aminoacid statine, of the formula

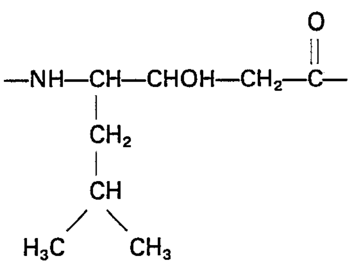

3S,4S isomer

and $Statyl_2$ denotes the statyl radical derived from the same aminoacid, but it can be the 3S,4S isomer or the 3R,4S isomer,

— R denotes a hydrogen atom or an acylating group attached to the terminal amino group of the aminoacid X,

— X and Y, which are identical or different, denote aminoacids of the L or D configuration when a centre of asymmetry exists in the molecule, and optionally protected in their side chains, said aminoacids being selected from the following aminoacids:

— X=Phenylalanine, Tryptophan, Histidine, Boc-Histidine, Tyrosine, Proline, Isoleucine;

— Y=Phenylalanine, Tryptophan, Histidine, Tyrosine, Proline, Leucine, Isoleucine, Valine, Glycine;

— R' represents OH, O-lower alkyl and the pharmaceutically acceptable salts of the products of the formula (I) when R' represents OH, characterized in that the starting material used is a lower alkyl ester of statine, from which the various aminoacids, suitably protected, are stepwise coupled the product obtained each step being freed, in accordance with known processes, before being subjected to a further coupling, and each of the coupling operations being carried out using either an activated ester of the aminoacid to be coupled, or the N-protected aminoacid in the presence of dicyclohexylcarbodiimide.

2. Process according to claim 1, characterized in that the freeing of the product obtained is carried out by hydrogenolysis or by hydrolysis in a strong acid medium.

3. Process according to one of claims 1 and 2, characterized in that, at the end of the reaction, the lower alkyl ester group of the statine is saponified in an alkaline medium so as to give the products of the formula (I) in which R' is H.